# EUROPEAN PATENT SPECIFICATION

(11) **EP 0 413 455 B1**
(45) Date of publication and mention of the grant of the patent: **21.06.1995**
(21) Application number: 90308331.9
(22) Date of filing: 30.07.1990
(51) Int. Cl.: C07D 401/04, C07D 471/04, C07D 498/06, A61K 31/435, A61K 31/47, A61K 31/535, C07D 209/52

(54) **Azabicyclo quinolone carboxylic acids**
Azabicyclo-Chinolin-Carbonsäuren
Acides azabicyclo quinoline carboxyliques

(30) Priority: 16.08.1989 WO PCT/US89/03489
(43) Date of publication of application: 20.02.1991
(73) Proprietor: PFIZER INC., New York, N.Y. 10017 (US)
(72) Inventor: Brighty, Katherine Elizabeth, Groton, Connecticut (US)
(74) Representative: Wood, David John

(56) References cited:
- CHEMICAL ABSTRACTS, vol. 111, 1989, page 221, abstract no. 153779w, Columbus,Ohio, US; & JP-A-01 56 673 (DAINIPPON PHARMACEUTICAL CO., LTD) 03-03-1989
- ARZNEIMITTEL-FORSCHUNG, vol. 21, no. 12, 1971, pages 2089-2100, Aulendorf,DE; H. WOLLWEBER et al.: "Guanidinoalkyl-azacycloalkane: Chemische Struktur und antihypertensive Wirkung"

## Description

The invention relates to novel 7-azabicyclo-substituted quinolone carboxylic acids, pharmaceutical compositions containing such compounds and methods of treatment with such compounds.

U.S. Patent 4,571,396 discloses diazabicyclo-substituted naphthyridine-, quinoline- and benzoxazinecarboxylic acids having antibacterial activity. European Patent Publication No. 215650 discloses similar antibacterial diazabicyclo-substituted compounds.

The invention provides antibacterial compounds having the formula 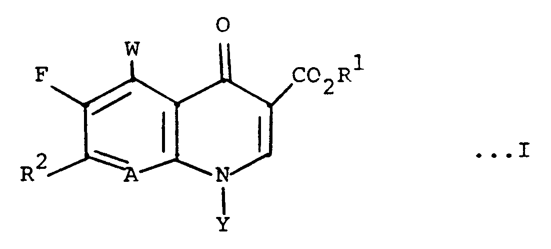 or a pharmaceutically acceptable acid addition salt thereof, wherein
R¹ is hydrogen, a pharmaceutically acceptable cation, or (C₁-C₆) alkyl;
Y, when taken independently, is ethyl, t-butyl, vinyl, cyclopropyl, 2-fluoroethyl, p-fluorophenyl, or o,p-difluorophenyl;
W is hydrogen, F, Cl, Br, C₁-C₄ alkyl, C₁-C₄ alkoxy, NH₂, NHCH₃;
A is CH, CF, CCl, COCH₃, C-CH₃, C-CN or N; or
A is carbon and is taken together with Y and the carbon and nitrogen to which A and Y are attached to form a five or six membered ring which may contain oxygen or a double bond, and which may have attached thereto R⁸ which is methyl; and
R² is 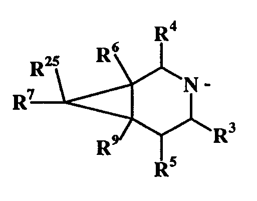 wherein R³, R⁴, R⁵, R⁶, R⁷, R⁹ and R²⁵ are each independently H, CH₃, CH₂NH₂, CH₂NHCH₃ or CH₂NHC₂H₅, and R⁵, R⁶, R⁷ and R⁹ may also independently be NH₂, NHCH₃ or NHC₂H₅, provided that not more than three of R³, R⁴, R⁵, R⁶, R⁷, R⁹ and R²⁵ are other than hydrogen, and if three of these substituents are not hydrogen, at least one of them is methyl; or wherein R¹ is H or a pharmaceutically acceptable cation, Y is 0,p-difluorophenyl, W is H, A is N and R² is 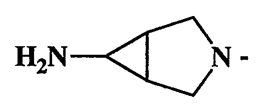 and prodrugs of those compounds of formula I having a free amino group.

Preferred compounds of the invention are those of formula I therein R¹ is hydrogen or a pharmaceutically acceptable cation such as sodium or potassium, and hydrates thereof. Other preferred compounds are the p-toluene-sulfonate, methanesulfonate and hydrochloride salts of the compounds of formula I.

Other preferred compounds are those wherein A is CH or N, or A is carbon and is taken together with Y and the carbon and nitrogen to which A and Y are attached to form a six membered ring as follows: 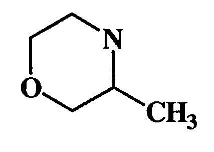 More preferably, A is CH or N, and most preferably, A is N. More specific compounds are those wherein one or two of R³, R⁴, R⁵, R⁶, R⁷, R⁹ and R²⁵ are other than hydrogen. Further jmore specific compounds are those wherein one of R³, R⁴, R⁵, R⁶ or R⁷, is CH₂NH₂ or CH₂NHCH₃, and, optionally, another of R³, R⁴, R⁵, R⁶, R⁷ or R⁹, is methyl; or those wherein one of R⁵, R⁶, R⁷ or R⁹ is NH₂ or NHCH₃ and, optionally, another of R⁵, R⁶, R⁷ or R⁹ or one of R³ and R⁴ is methyl rather than hydrogen. Preferred are those wherein R⁶, R⁷, or R⁹ is amino and, optionally one of R³, R⁴, R⁵, R⁶, R⁷ or R⁹ is methyl, and more preferred R⁷ is amino and, optionally, one of R³, R⁴, R⁵, R⁶, R⁷ and R⁹ is methyl. In the most preferred compounds R⁷ is amino and R³, R⁴, R⁵, R⁶, R⁷ and R⁹ are each hydrogen.

Other preferred compounds are those of formula I wherein Y is cyclopropyl or o,p-difluorophenyl, and those wherein W is hydrogen.

Specific compounds of the invention are
7-([1α,6α,7α]-7-amino-3-azabicyclo[4.1.0]hept-3-yl)-6-fluoro-1-(2,4-difluorophenyl)-1,4-dihydro-4-oxo-1,8-naphthyridine-3-carboxylic acid,
7-([1α,6α,7α]-7-amino-3-azabicyclo[4.1.0]hept-3-yl)-1-cyclopropyl-6-fluoro-1,4-dihydro-4-oxo-1,8-naphthyridine-3-carboxylic acid,
7-(1-amino-3-azabicyclo[4.1.0]hept-3-yl)-1-cyclopropyl-6-fluoro-1,4-dihydro-4-oxo-quinoline-3-carboxylic acid,
7-[(1α,5α,6α)-6-amino-3-azabicyclo[3.1.0]hex-3-yl]-6-fluoro-1-(2,4-difluorophenyl)-1,4-dihydro-4-oxo-1,8-naphthyridine-3-carboxylic acid,
7-[(1α,5α,6α)-6-amino-3-azabicyclo[3.1.0]hex-3-yl]-6-fluoro-1-(2,4-difluorophenyl)-1,4-dihydro-4-oxo-1,8-naphthyridine-3-carboxylic acid, hydrate,
7-([1α,5α,6α]-6-amino-3-azabicyclo[3.1.0]hex-3-yl)-6-fluoro-1-(2,4-difluorophenyl)-1,4-dihydro-4-oxo-1,8-naphthyridine-3-carboxylic acid, hydrochloride salt, methanesulfonic acid salt,
7-([1α,5α,6β]-6-amino-3-azabicyclo[3.1.0]hex-3-yl)-6-fluoro-1-(2,4-difluorophenyl)-1,4-dihydro-4-oxo-1,8-naphthyridine-3-carboxylic acid, and
The compounds of formula I of the invention wherein R³, R⁴, R⁵, R⁷, and R²⁵ are other than hydrogen can bear these substituents in either of two steric configurations relative to the cyclopropyl group in R². The compounds of formula I of the invention include the racemic mixtures and the optical isomers of all of these configurations.

The invention includes prodrugs of compounds of the formula I having free amino groups. Prodrugs are understood to comprise an amino acid residue, or a polypeptide chain of two or more amino acid residues which are covalently joined through peptide bonds. The amino acid residues of use include the 20 naturally occurring amino acids designated by three letter symbols, 4-hydroxyproline, hydroxylysine, demosine, isodemosine, 3-methylhistidine, norvalin, beta-alamine, gamma-aminobutyric acid, citrulline homocysteine, homoserine, ornithine and methionine sulfone. Preferred amino acid residues are those with a non-polar group such as Ala, Val, Nval, Leu, Met, Gly, Pro, Phe, or a basic polar group such as Lys.

The invention includes a pharmaceutical composition comprising a pharmaceutically acceptable carrier or diluent and a compound of the formula I in an antibacterially effective amount.

The invention further includes a method of treating a host, such as an animal or a human being, having a bacterial infection comprising administering to the host an antibacterially effective amount of a compound of the formula I, or a pharmaceutical composition as defined above.

The invention also includes intermediates of use in the preparation of a compound of the formula I. Exemplary intermediates have the formula 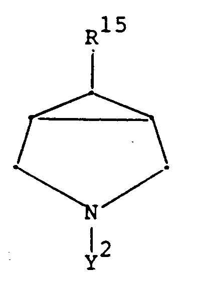 wherein Y² is hydrogen, benzyl, or benzyloxycarbonyl, and
R¹⁵ is carboxyl, hydroxymethyl, CHO, CH₂NR¹¹R¹² or NR¹¹R¹² wherein R¹¹ is hydrogen, methyl, or ethyl, and R¹² is hydrogen, C₁-C₆ acyl, C₂-C₆ alkoxycarbonyl, optionally substituted benzyloxycarbonyl, aryloxycarbonyl, silyl, trityl, tetrahydropyranyl, vinyloxycarbonyl, o-nitrophenylsulfonyl, diphenylphosphonyl, p-toluenesulfonyl, or benzyl.

Other intermediates of use in preparing compounds I are evident from the description below, particularly the sections numbered by Roman numerals.

The term "C₁-C₆ alkyl", used in the definition of R¹, denotes saturated monovalent straight or branched aliphatic hydrocarbon radicals having one to six carbon atoms, such as methyl, ethyl, propyl, isopropyl, t-butyl, etc.

When A is carbon and is taken together with Y and the carbon and nitrogen to which A and Y, respectively, are attached to form a five membered ring or a six membered ring, the compounds of formula I in one specific embodiment have the following formula: 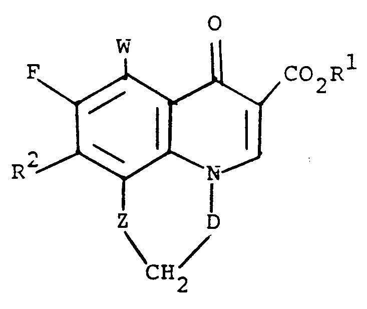 wherein Z is CH₂, O or a covalent bond, and D is CH₂, CHCH₃ or C=CH₂, and D may be CH=CH when Z is a covalent bond.

The compounds (I) of the invention may be prepared by reacting a compound of the formula 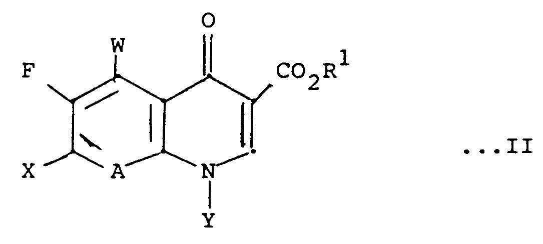 with a compound of the formula R²H wherein R¹, R², A, W and Y are as defined above in connection with formula I, except that R² includes within the definitions of R³, R⁴, R⁵, R⁶, R⁷ and R⁹, the N-protected groups of NH₂, CH₂NH₂, NHCH₃, CH₂NHCH₃, NHC₂H₅, and CH₂NHC₂H₅, and X is a leaving group such as fluoro, chloro, bromo or C₁-C₃ alkylsulfonyl. Nitrogen protecting groups are known in the art. Examples of suitable nitrogen protecting groups are C₁-C₆ acyl, C₂-C₆ alkoxycarbonyl optionally substituted benzyloxycarbonyl, aryloxycarbonyl, silyl, trityl, tetrahydropyranyl, vinyloxycarbonyl, 0-nitrophenylsulfonyl, diphenylphosphinyl, p-toluenesulfonyl, and benzyl. The nitrogen protecting group is removed by methods known in the art such as hydrogenation or hydrolysis.

The reaction may be conducted with or without a solvent. The solvent, when used, must be inert under the reaction conditions. Suitable solvents are acetonitrile, tetrahydrofuran, ethanol, chloroform, dimethylsulfoxide, dimethylformamide, pyridine, water, or mixtures thereof.

The reaction temperature usually ranges from about 20°C to about 150°C.

The reaction may advantageously be carried out in the presence of an acid acceptor such as an inorganic or organic base, e.g. an alkali metal or alkaline earth metal carbonate or bicarbonate, or a tertiary amine, e.g. triethylamine, pyridine or picoline.

When R¹ is C₁-C₆ alkyl, conversion to the corresponding acid may be carried out under acidic or basic conditions conventional for hydrolysis of carboxylic acid esters, at about 20° to 150°C.

The starting materials of formula II are known in the art, e.g. as disclosed in U.S. Patents 4,571,396 and 4,775,668. The starting materials of formula R²H have the following formulae 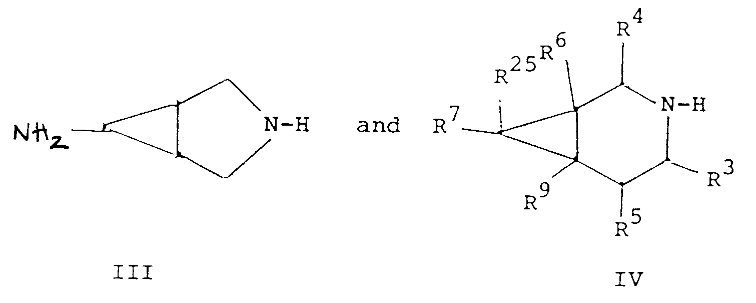 wherein R³, R⁴, R⁵, R⁶, R⁷, R⁹, R¹⁰ and R²⁵ are as defined above in connection with a compound of the formula R²H. Specific examples of such and similar starting materials are the following compounds: 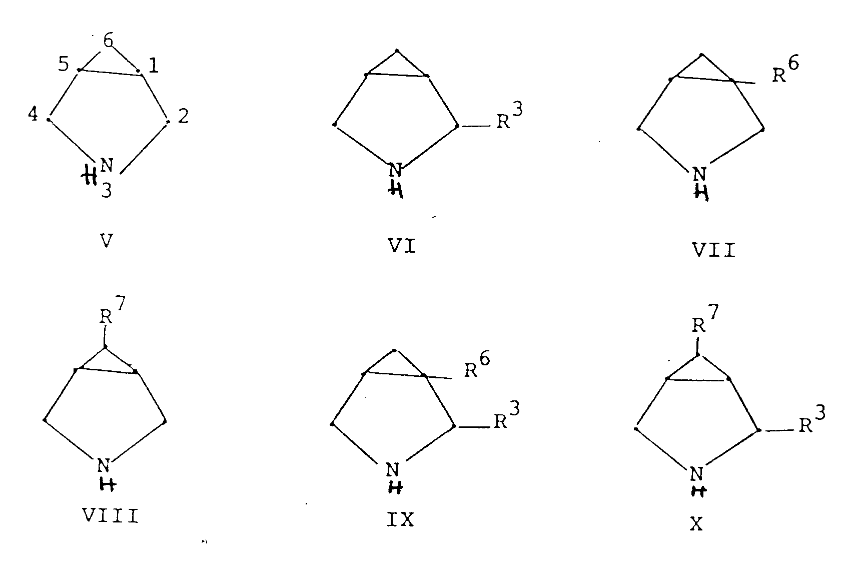 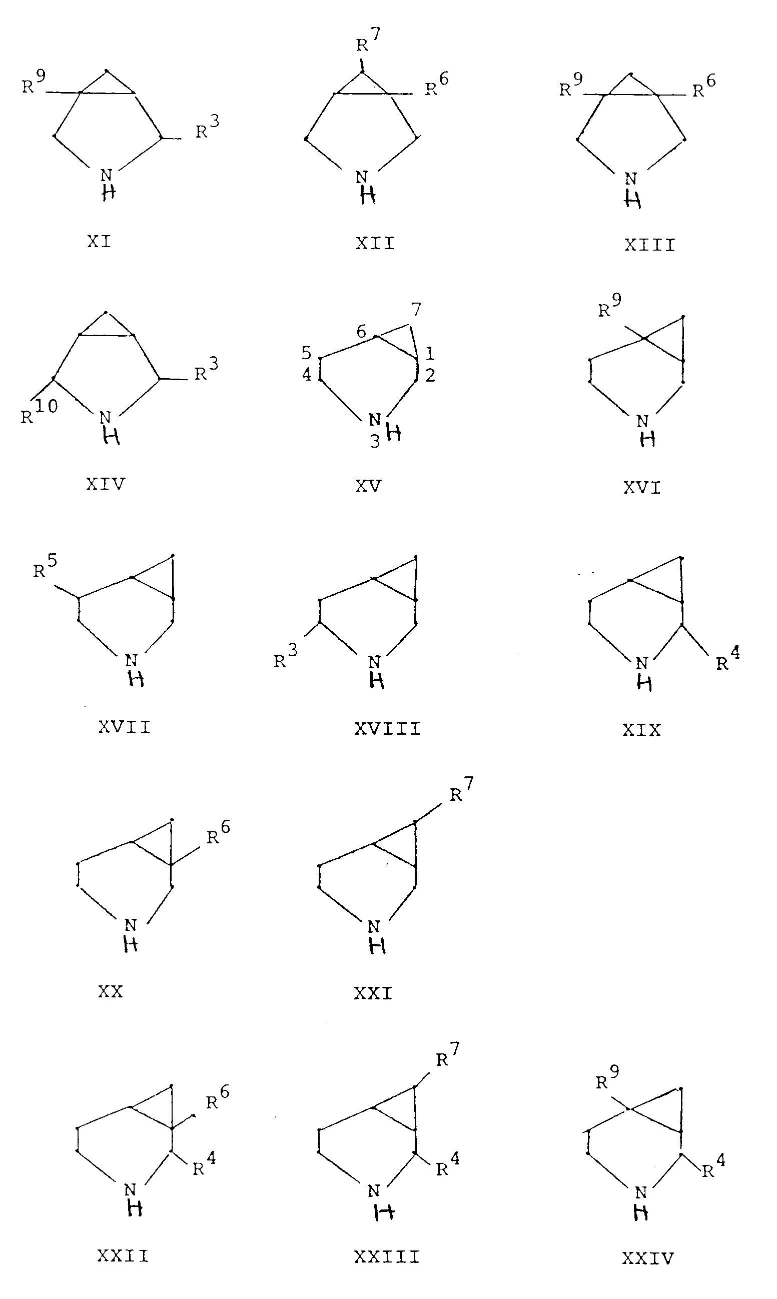 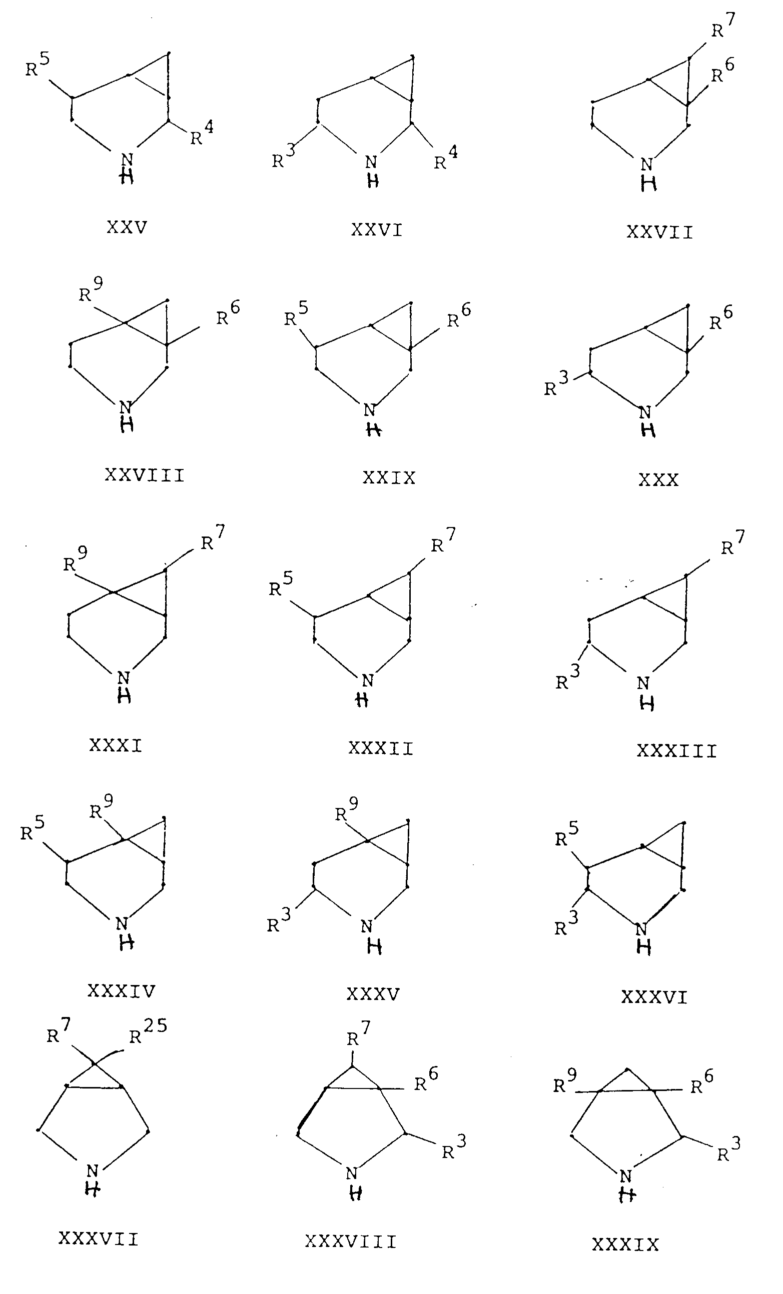 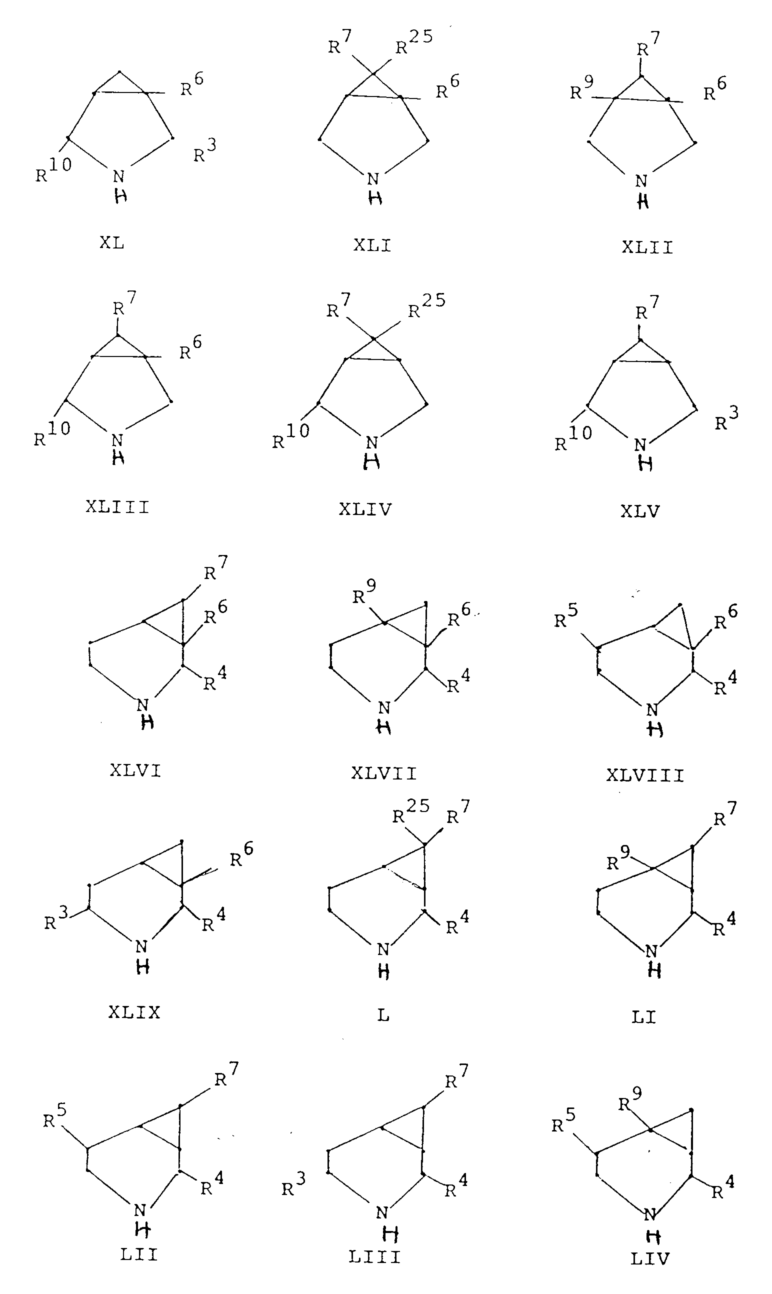 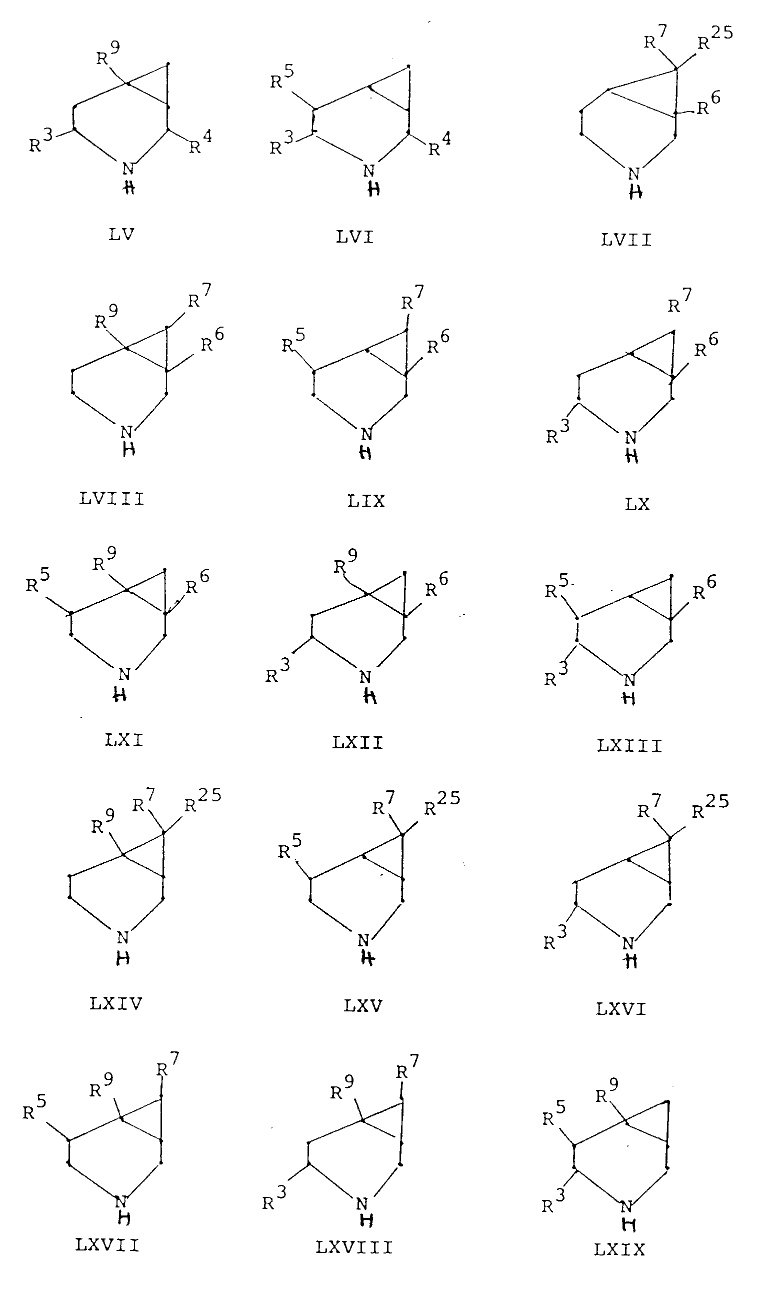 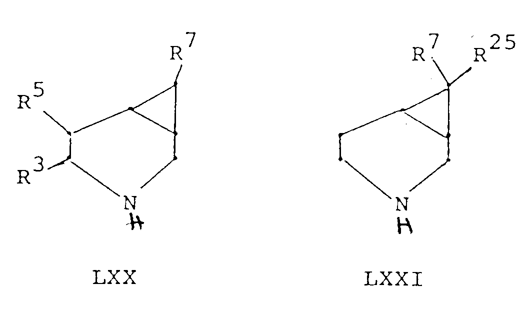 wherein R³, R⁴, R⁵, R⁶, R⁷, R⁹, and R²⁵ are as defined above, except hydrogen, and R¹⁰ is as defined as for R⁵.

The preparation of representative foregoing compounds I to XXI is discussed below wherein each section is referred to by the formula of the compounds prepared.

### 3-Azabicyclo[3.1.0]hexane (V)

3-Azabicyclo[3.1.0]hexane may be prepared by the method of D. A. Wood et al. European Patent Publication 0010799 from 1,2-cyclopropanedicarboxylic acid.

### 2-R³-Substituted 3-Azabicyclo[3.1.0]hexanes (VI)

2-Cyano-3-azabicyclo[3.1.0]hexane can be prepared by the method of D. A. Wood et al. EP 0010799. Protection of the ring nitrogen, for instance by a benzyl group, then provides 3-benzyl-2-cyano-3-azabicyclo[3.1.0]hexane. Reduction of the nitrile with lithium aluminum hydride gives a compound of the formula VI wherein R³ is CH₂NH₂ and the 3-N is benzylated. This compound, and all subsequently described amino-substituted azabicyclo[3.1.0]hexyl systems, may be advantageously protected, for instance with an alkoxycarbonyl group such as tert-butoxy-carbonyl, or a carboxylic acid group such as formyl or acetyl, and subsequently debenzylated via hydrogenation to provide the protected 2-aminomethyl-3-azabicyclo[3.1.0]hexane. After coupling of this debenzylated diamine to a quinolone or naphthyridine nucleus by reaction with a compound of the formula II, the amino-protecting group such as the tert-butoxy-carbonyl or acetyl group can be removed by exposure to acidic conditions.

Alternatively, the diamine 2-aminomethyl-3-benzyl-3-azabicyclo[3.1.0]hexane can be formylated or acetylated by heating to reflux with ethyl formate, according to the procedure of Moffat et al., J. Org. Chem., 27, 4058 (1962), or acetyl chloride. These amides can then be reduced to the corresponding amines with lithium aluminum hydride, to provide a compound of the formula VI wherein R³ is CH₂NHCH₃ or CH₂NHC₂H₅. This compound may be protected, as in the case of the conversion of the above diamine 2-aminomethyl-3-benzyl-3-azabicyclo[3.1.0]hexane to 2-[(N-acetyl)aminomethyl] or 2-[(N-tert-butoxycarbonyl)aminomethyl]-3-benzyl-3-azabicyclo[3.1.0]hexane, then debenzylated and appended to the quinolone or naphthyridine nucleus by reaction with a compound of the formula II.

For the case wherein R³ is CH₃, the above nitrile 3-benzyl-2-cyano-3-azabicyclo[3.1.0]hexane can be hydrolyzed under acidic or basic conditions to the corresponding carboxylic acid, and reduced with lithium aluminum hydride to the alcohol 3-benzyl-2-hydroxymethyl-3-azabicyclo[3.1.0]hexane. Formation of the tosylate followed again by lithium aluminum hydride reduction provides the 2-methyl congener 3-benzyl-2-methyl-3-azabicyclo[3.1.0]hexane, which can be debenzylated as above.

### 1-R⁶-Substituted-3-azabicyclo[3.1.0]hexanes (VII)

These compounds can be prepared from the nitrile 3-benzyl-1-cyano-3-azabicyclo[3.1.0]hexane, whose preparation is reported by Achini and Oppolzer, Tetrahedron Letters, 1975, 369. Alternatively, the nitrile may be synthesized from 3-[(benzyl)(2,3-dihydroxypropyl)amino]-propanenitrile via bismesylation, followed by double ring closure with sodium hexamethyldisilazide. Transformation of the nitrile functionality of 3-benzyl-1-cyano-3-azabicyclo[3.1.0]hexane into CH₃, CH₂NH₂, CH₂NHCH₃ or CH₂NHC₂H₅ can be carried out as in section VI above.

Hydrolysis of 3-benzyl-1-cyano-3-azabicyclo[3.1.0]hexane to 3-benzyl-3-azabicyclo[3.1.0]hexane-1-carboxylic acid can be carried out under basic conditions. Subsequent reaction with diphenylphosphoryl azide in t-butanol, using the procedure reported by Ninomiya et al., Tetrahedron 1974, 30, 2151, provides the protected amine 3-benzyl-1-tert-butoxycarbonylamino-3-azabicyclo[3.1.0]hexane. Debenzylation as above yields an amine which can be coupled to the quinolone or naphthyridine nucleus by reaction with a compound of the formula II; acidic removal of the tert-butoxycarbonyl group provides the final product with an amino group as the 1-substituent in the 3-azabicyclo[3.1.0]hexane side chain.

Removal of the tert-butoxycarbonyl group from the protected amine to give 1-amino-3-benzyl-3-azabicyclo[3.1.0]hexane can be followed by acetylation or formylation and lithium aluminum hydride reduction as above to provide a compound of the formula VII wherein R⁶ is NHCH₃ or NHC₂H₅. This can be further processed as in Section VI to provide the final product bearing a methylamine or ethylamine at C-1 of the 3-azabicyclo[3.1.0]hexane side chain.

Alternatively, 3-benzyl-1-tert-butoxycarbonylamino-3-azabicyclo[3.1.0]hexane can be N-alkylated by treatment with sodium hydride and methyl or ethyl iodide. The resulting diprotected N-alkyl compound can be debenzylated and processed as in Section VI.

### 6-R⁷-Substituted-3-azabicyclo[3.1.0]hexanes (VIII)

Addition of ethyl diazoacetate to N-benzylmaleimide generates a pyrazoline which upon thermolysis provides 3-benzyl-3-azabicyclo[3.1.0]hexane-2,4-dione-6-carboxylic acid ethyl ester. Reduction with lithium aluminum hydride gives 3-benzyl-6-hydroxymethyl-3-azabicyclo[3.1.0]hexane; Swern oxidation followed by oxime formation and lithium aluminum hydride reduction then produces the primary amine, which can be protected or treated as above to give a compound of formula VIII wherein R⁷ is CH₂NHCH₃ or CH₂NHCH₂CH₃.

Alternatively, 3-benzyl-6-hydroxymethyl-3-azabicyclo[3.1.0]hexane can be treated as in Section VI to provide the 6-methyl derivative. To prepare compounds with a 6-amino group, hydrogenolytic removal of the benzyl group from 3-benzyl-6-hydroxymethyl-3-azabicyclo[3.1.0]hexane is followed by introduction of a benzyloxycarbonyl group; Jones oxidation at this point provides 3-benzyloxycarbonyl-3-azabicyclo[3.1.0]hexane-6-carboxylic acid. Curtius rearrangement as in Section VII, using diphenylphosphoryl azide, yields 3-benzyloxycarbonyl-6-tert-butoxycarbonylamino-3-azabicyclo[3.1.0]hexane, which can be taken on to the analogue bearing a primary amine, or which can be deprotected and further manipulated as in Section VII to provide the compounds of formula VIII wherein R⁷ is NHCH₃ or NHC₂H₅.

Another route to these compounds involves treatment of N-benzyloxycarbonyl-3-pyrroline with ethyl diazoacetate under rhodium acetate catalysis, to provide the ethyl ester of 3-benzyloxycarbonyl-3-azabicyclo[3.1.0]hexane-6-carboxylic acid. Basic hydrolysis, for instance with sodium hydroxide in methanol, then gives the corresponding carboxylic acid, which can be processed as described above. Alternatively, the benzyloxycarbonyl group can be removed by hydrogenolysis, and the nitrogen functionality protected as a benzyl derivative, by treatment with benzyl bromide. Subsequent lithium aluminum hydride reduction then gives 3-benzyl-6-hydroxymethyl-3-azabicyclo[3.1.0]hexane, which can be further functionalized as described above.

### 1,2-R⁶,R³-Disubstituted-3-azabicyclo[3.1.0]hexanes (IX)

Modification of the Oppolzer procedure mentioned in Section VII provides this substitution pattern. For the 2-methyl substituted compounds, 3-benzylaminobutanenitrile is used as the starting material. For all other 2-substituents, 3-(benzylamino)-4-[(tetrahydro-2H-pyran-2-yl)oxy]-butanenitrile, available from beta-cyanoalanine via carboxylic acid reduction, alcohol protection and N-benzylation, can be reacted with glycidol to provide 3-[(benzyl)(2,3-dihydroxy-propyl)amino]-4-[(tetrahydro-2H-pyran-2-yl)oxy]butanenitrile. Tosylation of the primary alcohol is followed by base-induced ring closure to 3-[(benzyl) (2,3-epoxypropyl)amino]-4-[(tetrahydro-2H-pyran-2yl)oxy]-butanenitrile; sodium hexamethyldisilazide treatment provides 1-benzyl-4-hydroxymethyl-2-[(tetrahydro-2H-pyran-2-yl)oxy]methyl-3-pyrrolidine-carbonitrile. A second tosylation can be followed again by base-induced ring closure to the 3-azabicyclo[3.1.0]hexane of the formula IX wherein the 2-substituent is tetrahydropyranyloxymethyl, the 1-substituent is cyano, and the 3-aza nitrogen is benzylated. The nitrile functionality of the latter can be transformed into all of the substituents R⁶ as in Section VII.

For the elaboration of the C-2 substituent R³, final C-1 substituents R⁶ bearing amino groups can be protected as the corresponding acetamides. Subsequent acid-induced removal of the tetrahydropyran (THP) protecting group gives a primary alcohol which can be subjected to a Swern oxidation; reductive amination of the derived aldehyde with ammonium acetate, methylamine or ethylamine then provides the corresponding amines of the formula IX wherein R⁶ is CH₃ or amino-protected CH₂NH₂, CH₂NHCH₃, CH₂NHC₂H₅, NH₂, NHCH₃, or NHC₂H₅, and R³ is CH₂NH₂, CH₂NHCH₃, or CH₂NHC₂H₅. Protection of the resultant 2-amine can be carried out as above, with the tert-butoxycarbonyl protecting group; removal of the benzyl group via hydrogenation provides the free secondary amine, which can te coupled to the quinolone or naphthyridine nucleus, followed by acid-induced removal of the acetamide and tert-butoxycarbonyl groups.

### 2,6-R³,R⁷-Disubstituted-3-azabicyclo[3.1.0]hexanes (X)

These compounds can be prepared from 3-benzyl-6-hydroxymethyl-3-azabicyclo[3.1.0]hexane; protection as the THP ether, followed by debenzylation, provides 6-[(tetrahydro-2H-pyran-2-yl)oxy]methyl-3-azabicyclo[3.1.0]hexane. A cyano group can then be introduced into the 2-position by the method of Wood, as in Section VI. Reintroduction of the benzyl group provides 3-benzyl-2-cyano-6-[(tetrahydro-2H-pyran-2-yl)oxy]methyl-3-azabicyclo[3.1.0]hexane, wherein the two substituents are differentially functionalized. The cyano group can be transformed into the desired 2-substituents, as described in Section VI. At this point, protection of any primary or secondary amine as its acetamide can be followed by acidic removal of the tetrahydropyran protecting group, and elaboration of the primary alcohol into the desired substituent by the methods outlined in Section VIII.

When the 6-substituent is a methyl group, elaboration of the tetrahydropyranyl ether is carried out prior to introduction of the cyano group at C-2. When the 2-substitutent is a methyl group, an alternate route involves rhodium acetate-catalyzed cyclopropanation of N-benzyloxycarbonyl-2-methyl-3-pyrroline (available via the chemistry described by Takano, Heterocycles, 1989, 29, 1861, starting with 4-hydroxy-1-pentene) with ethyl diazoacetate. The ester group can then be elaborated to the desired 6-substituent as in Section VIII.

### 1,4-R⁹,R³ Disubstituted-3-azabicyclo[3.1.0]hexanes (XI)

These compounds can be prepared from methyl acrylate and 2-benzylamino-3-[(tetrahydro-2H-pyran-2-yl)oxy]propanoic acid methyl ester; heating these reagents in methanol provides an adduct which can be cyclized with sodium hexamethyldisilazide to 1-benzyl-4-oxo-5-[(tetrahydro-2H-pyran-2-yl)oxy]methyl-3-pyrrolidine carboxylic acid methyl ester. Reduction and benzyl group removal is effected with Raney nickel; introduction of a benzyloxycarbonyl group is then followed by mesylation of the secondary alcohol and diazabicyclononane-mediated dehydration to give 1-benzyloxycarbonyl-2,5-dihydro-5-[(tetrahydro-2H-pyran-2-yl)oxy]methyl-1H-pyrrole-3-carboxylic acid, methyl ester. Cyclopropanation with diiodomethane and zinc/silver couple, according to the method of Denis et al., Synthesis, 1972, 549, gives the bicyclo[3.1.0]hexyl system of formula XI wherein the 1-substituent is CO₂CH₃, the 4-substituent is tetrahydropyranyloxymethyl, and the 3-nitrogen is protected with benzyloxycarbonyl. The ester can be reduced to the corresponding alcohol wherein the 1-substituent is hydroxymethyl with lithium borohydride. Removal of the benzyloxycarbonyl group by hydrogenolysis using 10% palladium on carbon can then be followed by benzylation with benzyl bromide, to provide the compound of formula XI wherein the 1-substituent is hydroxymethyl, the 4-substituent is tetrahydropyranyloxymethyl, and the 3-nitrogen is protected with benzyl. Alternatively, the cyclopropanation product obtained above can be hydrolyzed with sodium hydroxide to the corresponding acid wherein the 1-substituent is CO₂H. These two compounds can be manipulated as in Section VIII to provide the desired 1-substituent R⁹; after protection of the 1-substituent, the 4-substituent R³ can be generated from the tetrahydropyranyl-protected alcohol as in Section IX. Removal of the 3-benzyloxycarbonyl group can then be effected by hydrogenation.

When the desired 4-substituent is a methyl group, the chemistry described above can be carried out starting with 2-benzylamino-propanoic acid methyl ester.

### 1,6-R⁶,R⁷-Disubstituted-3-azabicyclo[3.1.0]hexanes (XII)

These compounds can be prepared from tert-butyl acrylate and N-benzylglycine methyl ester; 1-benzyloxycarbonyl-2,5-dihydro-1H-pyrrole-3-carboxylic acid, tert-butyl ester is then synthesized via the methods described in Section XI. Molybdenum hexacarbonyl-mediated cyclopropanation with ethyl diazoacetate then provides the bicyclic system of the formula XII wherein the 1-substituent is t-butyloxycarbonyl, the 6-substituent is ethyloxycarbonyl, and the 3-nitrogen is substituted by benzyloxycarbonyl. Selective hydrolysis of the tert-butyl ester with trifluoroacetic acid can be followed by diborane-mediated reduction of the liberated carboxylic acid and protection of the derived primary alcohol as its tetrahydropyranyl ether. The 6-carboethoxy group can then be transformed into the desired 6-substituent as described above with respect to compounds of the formula XI. For a 6-methyl substituent, the protecting group on nitrogen is changed from benzyloxycarbonyl to benzyl as outlined in Section IX. After protection of any primary or secondary amines, the tetrahydropyranyl group can be removed under acidic conditions and the primary alcohol can be elaborated into the desired 1-substituent by the methods outlined in Section VIII.

For the case of a 1-methyl substituent, N-benzyloxycarbonyl-3-methyl-3-pyrroline (available via N-protection of 3-methyl-3-pyrroline, whose preparation is described by Gajda, Liebigs Ann. Chem, 1986, 992) is cyclopropanated using ethyl diazoacetate under rhodium acetate catalysis, to give a compound of formula XII wherein the 1-substituent is methyl, the 3-substituent is benzyloxycarbonyl, and the 6-substituent is ethoxycarbonyl. The ester functionality is then elaborated as described above.

### 1,5-R⁶,R⁹-Disubstituted-3-azabicyclo[3.1.0]hexanes (XIII)

These compounds are derived from 1-benzyl-4-hydroxymethyl-3-pyrrolidine carbonitrile, whose preparation is described by Achini and Oppolzer as mentioned in Section VII. Protection of the primary alcohol followed by nitrile hydrolysis and diazomethane esterification provides 1-benzyl-4-[(tetrahydro-2H-pyran-2-yl)oxy]methyl-3-pyrrolidine carboxylic acid methyl ester. The benzyl group can be removed by hydrogenation and replaced by a benzyloxycarbonyl group. Introduction of a thiophenyl group can then be effected via deprotonation with sodium hydride and reaction of the derived enolate with S-phenyl benzenethiosulfonate to give 1-benzyloxycarbonyl-4-[(tetrahydro-2H-pyran-2-yl)oxy]methyl-3-thiophenyl-3-pyrrolidinecarboxylic acid methyl ester. Oxidation of the sulfur with hydrogen peroxide, followed by thermolysis of the derived sulfoxide then gives alkene 1-benzyloxycarbonyl-2,5-dihydro-4-[(tetrahydro-2H-pyran-2-yl)oxy)methyl-1H-pyrrol-3-carboxylic acid methyl ester. Cyclopropanation with diiodomethane provides the bicyclic system of formula XIII wherein the 1-substituent is methoxycarbonyl, the 5-substituent is tetrahydropyranyloxymethyl, and the 3-aza is substituted by benzyloxycarbonyl, which can be further elaborated as in Section XII to give all of the disubstituted compounds.

When the 1-substituent is methyl, the benzyloxycarbonyl group is replaced with a benzyl group, as in Section XI, prior to conversion of the tetrahydropyranyloxymethyl group to a methyl group.

### 2,4-R³,R¹⁰-Disubstituted 3-azabicyclo[3.1.0]hexanes (XIV)

These compounds can be prepared from 3-benzyl-2-hydroxymethyl-3-azabicyclo[3.1.0]hexane by protection of the primary alcohol as the tetrahydropyranyl ether, debenzylation, introduction of a cyano group at the 4-position, and conversion into the desired 2- and 4-substituents according to the methods described in Section X.

### 3-Azabicyclo[4.1.0]heptane (XV)

Reaction of 1-benzyl-1,2,5,6-tetrahydropyridine with diazomethane and zinc iodide, according to the method of Attia, Ind. J. Chem., 16B, 98 (1978) provides 3-benzyl-3-azabicyclo[4.1.0]heptane. Hydrogenolytic removal of the benzyl group gives 3-azabicyclo[4.1.0]heptane.

### 6-R⁹-Substituted 3-Azabicyclo[4.1.0]heptanes (XVI)

Reaction of 3-benzylamino-1,2-dihydroxypropane with 4-bromobutanenitrile provides 4-[(benzyl) (2,3-dihydroxypropyl)amino]butanenitrile. Processing of this compound as in Section VII provides 3-benzyl-6-cyano-3-azabicyclo[4.1.0]heptane. The nitrile group of this compound can be transformed into the desired 6-R⁹-substituents as described in Section VII.

Alternatively, methyl 1-benzyloxycarbonyl-1,2,5,6-tetrahydropyridine-4-carboxylate can be reduced with diisobutylaluminum hydride, to provide 1-benzyloxycarbonyl-4-hydroxymethyl-1,2,5,6-tetrahydropyridine. Cyclopropanation using samarium amalgam and iodochloromethane, then gives 3-benzyloxycarbonyl-6-hydroxymethyl-3-azabicyclo[4.1.0]heptane. The hydroxymethyl group can be transformed into the desired substituent by the methods outlined in Section VIII.

### 5-R⁵-Substituted 3-Azabicyclo[4.1.0]heptanes (XVII)

These compounds can be prepared from 3-azabicyclo[4.1.0]heptan-4-one, disclosed in U.S. Patent 4,262,124. Reaction with sodium hydride and benzyl bromide provides 3-benzyl-3-azabicyclo[4.1.0]heptan-4-one, which can be subjected to treatment with strong base, such as lithium hexamethyldisilazide, and then reacted with formaldehyde. Subsequent protection of the resulting primary alcohol as the tetrahydropyranyl ether gives 3-benzyl-5-[(tetrahydro-2H-pyran-2-yl)oxy]methyl-3-azabicyclo[4.1.0]heptan-4-one. Lithium aluminum hydride reduction then yields the bicyclic system of the formula XVII where the 5-substituent is tetrahydropyranyl-protected hydroxymethyl. This substituent, after acid-induced removal of the THP group, can be transformed into the desired 5-R⁵-substituent by utilizing the methods described in Section VIII.

Alternatively, when R⁵ is amino or substituted amino, the compounds may be prepared starting from 1-benzyloxycarbonyl-5-hydroxy-1,2,5,6-tetrahydropyridine. Samarium-promoted cyclopropanation, as in Section XVI, can then be followed by replacement of the benzyloxycarbonyl group by a benzyl group, as in Section VIII (the benzyl bromide step can be replaced by treatment with benzaldehyde/sodium cyanoborohydride), to give 3-benzyl-5-hydroxy-3-azabicyclo[4.1.0]heptane. A Swern oxidation provides the corresponding ketone, and subsequent treatment with hydroxylamine hydrochloride, followed by lithium aluminum hydride reduction of the derived oxime, then gives 3-benzyl-5-amino-3-azabicyclo[4.1.0]heptane. Protection of the primary amine as its tert-butoxycarbonyl derivative can then be followed, if desired, by introduction of an N-methyl or N-ethyl group, as in Section VII.

### 4-R³-Substituted-3-Azabicyclo[4.1.0]heptanes (XVIII)

These compounds can be prepared from 2-hydroxymethylpyridine by protection of the primary alcohol as the tetrahydropyranyl ether followed by reaction with benzyl iodide, and sodium borohydride reduction, according to the method reported by Sashida and Tsuchiya, Chem. Pharm. Bull., 32, 4600 (1984), to provide 1-benzyl-2-[(tetrahydro-2H-pyran-2-yl)oxy]methyl-1,2,3,6-tetrahydropyridine. Cyclopropanation with diazomethane/zinc iodide, according to the method of Attia in Section XV, then gives 3-benzyl-4-[(tetrahydro-2H-pyran-2-yl)oxy]methyl-3-azabicyclo[4.1.0]heptane. Acid-induced removal of the tetrahydropyranyl group can be followed by methods described in Section VIII to provide the desired 4-R³-substituent.

### 2-R⁴-Substituted-3-Azabicyclo[4.1.0]heptanes (XIX)

Compounds of this type may be prepared from bicyclo[3.1.0]hexan-3-one by deprotonation with strong base, such as lithium hexamethyldisilazide, followed by quenching of the derived enolate with formaldehyde and protection of the resulting primary alcohol as the tetrahydropyranyl ether to provide 2-[(tetrahydro-2H-pyran-2-yl)oxy]methyl-bicyclo[3.1.0]hexan-3-one. Beckmann rearrangement of this compound, via the corresponding oxime tosylate, provides 2-[(tetrahydro-2H-pyran-2-yl)oxy]methyl-3-azabicyclo[4.1.0]heptan-4-one. Reaction with sodium hydride and benzyl bromide, followed by reduction with lithium aluminum hydride, then gives 3-benzyl-2-[(tetrahydro-2H-pyran-2-yl)oxy]methyl-3-azabicyclo[4.1.0]heptane; the protected hydroxymethyl 2-substituent can be transformed into the desired 2-substituent utilizing the methods described in Section IX.

### 1-R⁶-Substituted-3-Azabicyclo[4.1.0]heptanes (XX)

These compounds can be prepared from methyl 1-benzyloxycarbonyl-1,2,5,6-tetrahydropyridine-3-carboxylate, using the methodology described in Section XVI to generate 3-benzyloxycarbonyl-1-hydroxymethyl-3-azabicyclo[4.1.0]heptane. The methodology described in Section VIII can be used to convert the hydroxymethyl group into the desired substituent. In this case, as well as others where the Curtius rearrangement is employed, good results may be obtained using the modified Curtius reaction described by Overman, Org. Synth. Coll. Volume VI, 95.

### 7-R⁷-Substituted-3-Azabicyclo[4.1.0]heptanes (XXI)

These compounds can be prepared from 1-benzyl-5,6-dihydro-2(1H)-pyridinone by reaction with ethyl diazoacetate with molybdenum hexacarbonyl catalyst to provide 3-benzyl-2-oxo-3-azabicyclo[4.1.0]heptane-7-carboxylic acid ethyl ester, which can be reduced with lithium aluminum hydride to provide 3-benzyl-7-hydroxymethyl-3-azabicyclo[4.1.0]heptane. Utilization of the methods in Section VIII then yields the desired 7-R⁷-substituent.

Alternatively, 1-benzyloxycarbonyl-1,2,5,6-tetrahydropyridine can be subjected to reaction with ethyl diazoacetate under rhodium acetate catalysis, to provide ethyl-3-benzyloxycarbonyl-3-azabicyclo[4.1.0]heptane-7-carboxylate. Ester hydrolysis with sodium hydroxide then provides the corresponding carboxylic acid, which can be converted as described in Section VIII to give amino or substituted amino derivatives.

### 2,7-R⁴,R⁷-Disubstituted-3-azabicyclo[4.1.0]heptanes (XXIII)

These compounds are derived from 1-methyl-2-tetrahydropyranyloxymethyl-1,2,5,6-tetrahydropyridine, which can be prepared from 2-(hydroxymethyl)pyridine using the procedures outlined in Section XVIII. Treatment of 1-methyl-2-tetrahydropyranyloxymethyl-1,2,5,6-tetrahydropyridine with α-chloroethyl chloroformate, followed by methanol, serves to remove the 1-methyl group; treatment of the secondary amine with benzyl chloroformate then yields 1-benzyloxycarbonyl-2-tetrahydropyranyloxymethyl-1,2,5,6-tetrahydropyridine. Cyclopropanation of this compound with ethyl diazoacetate in the presence of catalytic rhodium acetate gives ethyl 3-benzyloxycarbonyl-2-tetrahydropyranyloxymethyl-3-azabicyclo[4.1.0]heptane-7-carboxylate. This can be transformed into a compound with the desired substitution pattern, using the chemistry described in Section XI.

### 2,6-R⁴,R⁹-Disubstituted-3-azabicyclo[4.1.0]heptanes (XXIV)

These compounds can be prepared from methyl 1-benzyloxycarbonyl-1,2,5,6-tetrahydropyridine-4-carboxylate. Deprotonation with strong base, such as lithium diisopropylamide or lithium hexamethyldisilazide, can be followed by reaction with formaldehyde and protection of the resulting primary alcohol as its tetrahydropyranyl derivative, to give methyl 1-benzyloxycarbonyl-2-tetrahydropyranyloxymethyl-1,2,5,6-tetrahydropyridine-4-carboxylate. Processing of this compound using methodology described in Section XVI provides 3-benzyloxycarbonyl-6-hydroxymethyl-2-tetrahydropyranyloxymethyl-3-azabicyclo[4.1.0]heptane, which can be converted into the desired disubstituted compound using chemistry from Sections VIII and XI.

### 1,7-R⁶,R⁷-Disubstituted-3-azabicyclo[4.1.0]heptanes (XXVII)

Methyl 1-benzyloxycarbonyl-1,2,5,6-tetrahydropyridine-3-carboxylate can be reduced with diisobutylaluminum hydride, and the resulting primary alcohol protected as its tetrahydropyranyl derivative. Cyclopropanation with ethyl diazoacetate in the presence of rhodium acetate then yields the ethyl ester of 3-benzyloxycarbonyl-1-tetrahydropyranyloxymethyl-3-azabicyclo[4.1.0]heptane-7-carboxylic acid. Processing of this compound as in Section XII delivers the desired substitution.

Alternatively, tert-butyl 1-benzyloxycarbonyl-1,2,5,6-tetrahydropyridine-3-carboxylate can be cyclopropanated using ethyl diazoacetate under molybdenum hexacarbonyl catalysis, to give 1-tert-butyl 7-ethyl 3-benzyloxycarbonyl-3-azabicyclo[4.1.0]heptane-1,7-dicarboxylic acid. Application of chemistry described in Section XII can be used to synthesize the desired disubstituted compound.

### 1,6-R⁶,R⁹-Disubstituted-3-azabicyclo[4.1.0]heptanes(XXVIII)

Addition of benzylamine to 1-tetrahydropyranyloxy-3-buten-2-one, followed by Wittig olefination of the ketone with methyltriphenylphosphonium bromide and base, provides 4-benzylamino-2-methylene-1-(tetrahydropyranyloxy)butane. Amide formation with monoethyl malonate, using carbonyldiimidazole as a condensing agent then provides a dicarbonyl compound, which is subjected to diazo transfer using p-toluenesulfonyl azide or p-carboxyphenylsulfonyl azide under the influence of potassium t-butoxide or potassium hydride. Alternatively, the procedure of Koskinen, J. Chem. Soc, Chem. Commun., 1990, 652 can be utilized. The resulting diazo compound is treated with rhodium acetate in refluxing benzene, according to the procedure of Kametani, Chem. Pharm. Bull., 1985, 61, to provide the ethyl ester of 3-benzyl-2-oxo-6-tetrahydropyranyloxymethyl-3-azabicyclo[4.1.0]heptane-1-carboxylic acid. Lithium aluminum hydride reduction gives a compound of formula XXVIII wherein the 1-substituent is hydroxymethyl and the 6-substituent is tetrahydropyranyloxymethyl. This compound can be processed into the desired sidechain by the chemistry described in Sections XI and VIII.

### 1,5-R⁶,R⁵-Disubstituted-3-azabicyclo[4.1.0]heptanes (XXIX)

2-Ethenyl-1,3-propanediol can be prepared using the methodology of Meyer, Syn. Commun., 1986, 261. Monoprotection as the tetrahydropyranyl derivative can be followed by mesylation of the remaining primary alcohol, and displacement with benzylamine, to provide 4-(benzylamino)-3-tetrahydropyranyloxymethyl-1-butene. Amide formation with monoethyl malonate, diazo transfer and cyclization as in Section XXVIII then provides the ethyl ester of 3-benzyl-2-oxo-5-tetrahydropyranyloxymethyl-3-azabicyclo[4.1.0]heptane-1-carboxylic acid. Lithium aluminum hydride reduction gives 3-benzyl-1-hydroxymethyl-5-tetrahydropyranyloxymethyl-3-azabicyclo[4.1.0]heptane, which can be transformed into the desired substituent using the chemistry outlined in Sections XI and VIII.

### 5,7-R⁵,R⁷-Disubstituted-3-azabicyclo[4.1.0]heptanes (XXXII)

Cycloaddition of 5-tetrahydropyranyloxy-1,3-pentadiene with the benzyl ester of methylenecarbamic acid provides 1-benzyloxycarbonyl-3-tetrahydropyranyloxymethyl-1,2,3,6-tetrahydropyridine. Cyclopropanation with ethyl diazoacetate and rhodium acetate then gives the ethyl ester of 3-benzyloxycarbonyl-5-tetrahydropyranyloxymethyl-3-azabicyclo[4,1,0]heptane-7-carboxylic acid. Conversion into the desired disubstituted compound can then be carried out as described in Section XII.

### 5,6-R⁵,R⁹-Disubstituted-3-azabicyclo[4.1.0]heptanes (XXXIV)

Addition of allylamine to ethyl 4-chloroacetoacetate, followed by protection of the resulting secondary amine as its benzyloxycarbonyl derivative provides ethyl N-allyl-N-benzyloxycarbonyl-4-amino-3-oxo-butanoic acid. Diazo transfer and rhodium-mediated cyclization can then be carried out, as described in Section XXVIII, to provide ethyl 3-benzyloxycarbonyl-5-oxo-3-azabicyclo[4.1.0]heptane-6-carboxylate. Olefination with (methoxymethyl)triphenylphosphonium chloride and base, followed by mild acid hydrolysis, then gives ethyl 3-benzyloxycarbonyl-5-carboxaldehyde-3-azabicyclo[4.1.0]heptane-6-carboxylate. Oxidation of the aldehyde to a carboxylic acid can be carried out with sodium chlorite or tetra-n-butylammonium permanganate. The resulting compound of formula XXXIV, wherein R⁵ is a carboxylic acid and R⁹ is an ethyl ester, can be transformed into the desired disubstituted compound using the procedure outlined in Section XII.

### 6,6-R⁷,R²⁵-Disubstituted-3-azabicyclo[3.1.0]hexanes(XXXVII)

These compounds are derived from methyl tert-butyl 3-benzyloxycarbonyl-3-azabicyclo[3.1.0]hexane-6,6-dicarboxylic acid, which can be prepared by cyclopropanation of 1-benzyloxycarbonyl-3-pyrroline using the method of Ohishi, Synthesis, 1980, 690 or Peace and Wulfman, Synthesis, 1973, 137. Removal of the tert-butyl ester can be effected by brief treatment with trifluoroacetic acid; the liberated carboxylic acid can then be transformed into an amino group by the procedure of Baldwin, J. Chem. Soc, Chem, Commun., 1988, 775. The resulting compound of formula XXXVII, wherein the 3-substituent is benzyloxycarbonyl and the 6-substituents are amino and methoxycarbonyl, can then be protected as its tert-butoxycarbonyl derivative; alkylation of the amine, as in Section VII, can be carried out to provide the N-methyl and N-ethyl derivatives. Reduction of the ester functionality ith lithium borohydride gives the primary alcohol, which can be processed as in Section IX to give aminomethyl substituents.

When at least one of the 6-substituents is methyl, the carboxylic acid resulting from deprotection of the tert-butyl ester is reduced with diborane, to provide a compound of the formula XXXVII wherein the 3-substituent is benzyloxycarbonyl and the 6-substituents are hydroxymethyl and methoxycarbonyl. Replacement of the benzyloxycarbonyl group by a benzyl group, as in Section XI, is then followed by tosylation of the alcohol. Reduction with lithium aluminum hydride yields a compound of formula XXXVII wherein the 3-substituent is benzyl, and the 6-substituents are methyl and hydroxymethyl. The hydroxymethyl group can be transformed into the desired substituent by the methods outlined in Section VIII.

Alternatively, to generate compounds where at least one of the 6-substituents is methyl, methodology of Loozen, J. Org. Chem, 1976, 2965 can be employed. Thus, 1-benzyloxycarbonyl-3-pyrroline can be reacted with dibromocarbene, to provide 3-benzyloxycarbonyl-6,6-dibromo-3-azabicyclo[3.1.0]hexane. One of the bromines is replaced by methyl, using n-butyllithium and methyl iodide. The resulting compound is again subjected to metal-halogen exchange, using butyllithium at low temperature, and the anion is quenched with formaldehyde, to provide 3-benzyl-6-hydroxymethyl-6-methyl-3-azabicyclo[3.1.0]hexane. Formation of the initial gem-dibromocyclopropane can also be effected using phenyl(tribromomethyl)mercury. The hydroxymethyl group can be transformed into the desired substituent by the methods outlined in Section VIII.

To generate compounds in which both of the 6-substituents are aminomethyl derivatives, methyl tert-butyl 3-benzyloxycarbonyl-3-azabicyclo[3.1.0]hexane-6,6-dicarboxylic acid is once again deprotected with trifluoroacetic acid. The liberated carboxylic acid is condensed with ammonia, methylamine or ethylamine through the use of an activating agent such as dicyclohexylcarbodiimide or carbonyl diimidazole, to form the corresponding amide. The methyl ester is then hydrolyzed to the carboxylic acid under acidic or basic conditions, and a second amide is formed in similar fashion. The resulting compound of formula XXXVII wherein both 6-substituents are amides, optionally substituted with a methyl or ethyl group, is then transformed from the N-benzyloxycarbonyl derivative to the N-benzyl compound, as in Section XI. Subsequent reduction with lithium aluminum hydride provides the compound bearing two aminomethyl groups at the 6-position, which are optionally substituted with a methyl or an ethyl group. Protection as the di-tert-butoxycarbonyl derivative and removal of the benzyl group by hydrogenolysis provides the compound in a form appropriate for coupling to compound II.

### 1,2,6-R⁶,R³,R⁷-Trisubstituted-3-azabicyclo[3.1.0]hexanes (XXXVIII)

### A. R⁷ is a methyl group.

These compounds are derived from 1-benzylamino-2-butene, available from the reaction of benzylamine with 1-bromo-2-butene. Amide formation with monoethyl malonate, diazo transfer, and cyclization using rhodium acetate can be carried out as in Section XXVIII, to provide the ethyl ester of 3-benzyl-6-methyl-2-oxo-3-azabicyclo[3.1.0]hexane-1-carboxylic acid. Reduction with lithium borohydride and protection of the resulting hydroxymethyl group as its tetrahydropyranyl ether provides a compound of the formula XXVVIII where R³ is a double bond to oxygen, R⁶ is a tetrahydropyranyloxymethyl group, and R⁷ is a methyl group. Subjection of this compound to methyllithium followed by sodium cyanoborohydride, according to the work of Shibagaki, Heterocycles, 1986, 423, gives 3-benzyl-2,6-dimethyl-1-tetrahydropyranyloxymethyl-3-azabicyclo[3.1.0]hexane, wherein the 1-substituent can be elaborated as in Section X, to give compounds of the formula XXXVIII where R³ and R⁷ are methyl groups.

Alternatively, the amide functionality in the 1-tetrahydropyranyloxymethyl compound can be reduced to the carbinolamine with sodium bis(2-methoxyethoxy)aluminum hydride (Red-Al) at -78°C. Methylation of the alcohol functionality with methyl iodide can be followed by displacement with trimethylsilylcyanide to provide a compound of formula XXXVIII wherein R³ is cyano, R⁶ is tetrahydropyranyloxymethyl and R⁷ is methyl. The cyano group can be transformed at this point into the desired substituent by the methods outlined in Section VI. The 1-substituent is converted from the tetrahydropyranyloxymethyl substituent by the chemistry described in Section IX or Section X.

### B. R⁶ is a methyl group.

Reaction of 3-methyl-1,4-pentadiene with less than one equivalent of osmium tetroxide provides a diol, which can be mono-protected at the primary alcohol to give 2-hydroxy-3-methyl-1-tetrahydropyranyloxy-4-pentene. Submission of this compound to the chemistry described by Takano, Heterocycles 1989, 1861, yields 1-benzyloxycarbonyl-3-methyl-2-tetrahydropyranyloxymethyl-3-pyrroline. Cyclopropanation with ethyl diazoacetate under rhodium acetate catalysis provides a compound of the formula XXXVIII wherein R³ is tetrahydropyranyloxymethyl, R⁶ is methyl and R⁷ is ethoxycarbonyl. Hydrolysis of the ethyl ester under basic conditions provides a carboxylic acid as the 6-substituent; this can be transformed into an amine or an alkylated amine using the chemistry described in Section VIII. Alternatively, the benzyloxycarbonyl group can be replaced by a benzyl group, as in Section XI; the ester group can then be converted to an (alkyl)aminomethyl group as in Section VIII. After protection of any amine groups at the 6-position, the tetrahydropyranyloxymethyl group can be converted into the desired substituent using the chemistry in Section IX or X.

When both the 1- and 6-substituents are methyl, the same chemistry can be effected starting with 1-benzyloxycarbonyl-2,3-dimethyl-3-pyrroline.

### C. R³ is a methyl group.

In this case, the starting material is the tert-butyl ester of 1-benzyloxycarbonyl-2-methyl-3-pyrroline-3-carboxylic acid, obtainable from the chemistry described in Section XII, where tert-butyl crotonate is employed in place of tert-butyl acrylate. Cyclopropanation as above with ethyl diazoacetate provides a compound of the formula XXXVIII wherein R³ is methyl, R⁶ is tert-butoxycarbonyl, and R⁷ is ethoxycarbonyl. Trifluoroacetic acid can be used to hydrolyze the tert butyl ester; subsequent Curtius rearrangement with diphenylphosphoryl azide in tert-butanol provides a protected 1-amino substituent, which can be alkylated as in Section VIII if desired. Alternatively, the acid moiety at the 1-position can be reduced with diborane to provide a hydroxymethyl substituent, which can be elaborated as in Section VIII or IX. The ethyl ester at the 6-position is then either hydrolyzed under basic conditions and the resulting acid subjected to a similar Curtius rearrangement and further elaboration, or reduced to the hydroxymethyl group with lithium borohydride. The hydroxymethyl group can then be converted into the desired substituent by the chemistry described in Section IX.

### 1,6,6-R⁶,R⁷,R²⁵-Trisubstituted-3-azabicyclo[3.1.0]hexanes (XLI)

### A. R⁶ is a methyl group.

These compounds can be prepared from 1-benzyloxycarbonyl-3-methyl-3-pyrroline by cyclopropanation with tert-butyl methyl malonate or its diazo derivative, as outlined in Section XXXVII. The resulting tert-butyl methyl 3-benzyloxycarbonyl-1-methyl-3-azabicyclo[3.1.0]hexane-6,6-dicarboxylic acid can be further functionalized as described in Section XXXVII.

### B. R⁷ is a methyl group.

Compounds of this type are derived from 1-benzyloxycarbonyl-3-tetrahydropyranyloxymethyl-3-pyrroline. This starting material can be preparei from 1-benzyloxycarbonyl-3-pyrrolidinone by deprotonation with strong base, such as lithium hexamethyldisilazide, followed by quenching with formaldehyde. The free alcohol is protected as its tetrahydropyranyl derivative, and the ketone is reduced with sodium borohydride. Dehydration of the resulting alcohol with phosphorus oxychloride in pyridine gives the requisite starting material.

Cyclopropanation with ethyl diazoacetate under rhodium acetate catalysis provides the ethyl ester of 3-benzyloxycarbonyl-1-tetrahydropyranyloxymethyl-3-azabicyclo[3.1.0]hexane-6-carboxylic acid, which can be methylated at the 6-position by deprotonation with strong base such as potassium hydride or lithium hexamethyldisilazide, and reaction of the derived enolate with methyl iodide. The ester can then be hydrolyzed using sodium hydroxide in methanol, and the resulting carboxylic acid functionalized as desired, using the methods described in Sections XI or XXXVIII(c).

Alternatively, the pyrroline starting material can be cyclopropanated as in Section XXXVII, to provide t-butyl methyl 3-benzyloxycarbonyl-1-tetrahydropyranyloxymethyl-3-azabicyclo[3.1.0]hexane-6,6-dicarboxylic acid. This can be processed as in Section XXXVII to generate 3-benzyloxycarbonyl-6-hydroxymethyl-6-methyl-1-tetrahydropyranyloxymethyl-3-azabicyclo[3.1.0]hexane. Use of chemistry outlined in Section XI then gives the desired substitution pattern.

### 1,5,6-R⁶,R⁹,R⁷-Trisubstituted-3-azabicyclo[3.1.0]hexanes (XLII)

### A. R⁷ is a methyl group.

1-Bromo-2-tetrahydropyranyloxymethyl-2-butene can be reacted with benzylamine, and the resulting secondary amine condensed with the monoethyl ester of malonic ester, as described in Section XXVIII. Diazo transfer and intramolecular cyclopropanation, as described in Section XXVIII, then provides ethyl 3-benzyl-6-methyl-2-oxo-5-tetrahydropyranyloxymethyl-3-azabicyclo[3.1.0]hexane-1-carboxylic acid. Lithium aluminum hydride reduction gives a compound of formula XLII wherein the 1-substituent is hydroxymethyl, the 5-substituent is tetrahydropyranyloxymethyl, and the 6-substituent is methyl. This compound can be processed into the desired sidechain by utilizing the chemistry described in Sections XI and VIII.

### B. R⁶ is a methyl group.

These compounds are derived from 1-chloro-2-methyl-4-tetrahydropyranyloxy-2-butene, whose preparation has been described Schmid, Helv. Chim. Acta, 1982, 684. Processing of this compound as in Section A above provides 3-benzyl-1-hydroxymethyl-5-methyl-6-tetrahydropyranyloxymethyl-3-azabicyclo[3.1.0]hexane. This compound can also be transformed into the desired sidechain by utilizing the chemistry described in Sections XI and VIII.

### 2,4,6-R³,R¹⁰,R⁷-Trisubstituted-3-azabicyclo[3.1.0]hexanes (XLV)

### A. R⁷ is a methyl group.

To prepare compounds of this type, 3-benzyl-6-methyl-3-azabicyclo[3.1.0]hexane (a preparation for which is outlined in Section VIII) is transformed into 3-benzyl-2-cyano-6-methyl-3-azabicyclo[3.1.0]hexane by the method described in Section X. Subsequent hydrolysis of the nitrile under acidic or basic conditions can be followed by lithium aluminum hydride reduction and protection of the resulting primary alcohol as its tetrahydropyranyl derivative. Further functionalization can be carried out as in Section XIV to provide the desired substitution pattern.

### B. R³ is a methyl group.

These compounds are derived from 1-benzyloxycarbonyl-2-methyl-3-pyrroline. Cyclopropanation with ethyl diazoacetate, as described in Section X, can be followed by ester reduction with lithium borohydride, and protection of the resulting primary alcohol as its tetrahydropyranyl derivative, to provide 3-benzyloxycarbonyl-2-methyl-6-tetrahydropyranyloxymethyl-3-azabicyclo[3.1.0]hexane. Removal of the benzyloxycarbonyl group by hydrogenolysis can then be followed by the introduction of a cyano group at the 4-position. The 4-cyano-2-methyl-6-tetrahydropyranyloxymethyl-3-azabicyclo[3.1.0]hexane obtained in this way can then be converted to the desired trisubstituted 3-azabicyclo[3.1.0]hexane by the methods outlined in Section X.

### 1,2,7-R⁶,R⁴,R⁷-Trisubstituted-3-azabicyclo[4.1.0]heptanes (XLVI)

### A. R⁷ is a methyl group.

Reaction of benzylamine with 5-bromopent-2-ene gives 5-benzylamino-2-pentene, which can be condensed with the half-ester of malonic acid, as described in Section XXVIII. Subsequent diazo transfer and cycloaddition, according to Section XXVIII, provides ethyl 3-benzyloxycarbonyl-7-methyl-2-oxo-3-azabicyclo[4.1.0]heptane-1-carboxylate. Processing of this compound as in Section XXXVIII provides the desired trisubstituted compound.

### B. R⁶ is a methyl group.

Cycloaddition of the benzyl ester of methylenecarbamic acid with 3-methyl-5-tetrahydropyranyloxy-1,3-pentadiene yields 1-benzyloxycarbonyl-3-methyl-2-tetrahydropyranyloxymethyl-1,2,5,6-tetrahydropyridine. Cyclopropanation with ethyl diazoacetate, as describad above, then provides a compound of formula XLVI, where R⁷ is an ethyl ester, R⁶ is methyl, and R⁴ is tetrahydropyranyloxymethyl. This compound can be transformed into the desired trisubstituted sidechain using methodology described in Section XI.

### C. R⁴ is a methyl group.

Cycloaddition of the benzyl ester of methylenecarbamic acid with 3-tetrahydropyranyloxymethyl-1,3-pentadiene provides 1-benzyloxycarbonyl-2-methyl-3-tetrahydropyranyloxymethyl-1,2,5,6-tetrahydropyridine. Cyclopropanation with ethyl diazoacetate gives a compound of formula XLVI, wherein R⁷ is an ethyl ester group, R⁶ is tetrahydropyranyloxymethyl, and R⁴ is methyl. Chemistry described in Section XII can be used to transform this compound into the desired sidechain.

### 2,7,7-R⁴,R⁷,R²⁵-Trisubstituted-3-azabicyclo[4.1.0]heptanes (L)

### A. R⁴ is a methyl group.

Cycloaddition of the benzyl ester of methylenecarbamic acid with 1,3-pentadiene provides 1-benzyloxycarbonyl-2-methyl-1,2,5,6-tetrahydropyridine. Cyclopropanation with tert-butyl methyl malonate or its diazo derivative, as outlined in Section XXXVII, then gives a compound of formula L wherein R⁴ is a methyl group, R⁷ is a methyl ester group and R²⁵ is a tert-butyl ester group. Chemistry outlined in Section XXXVII is then used to convert this compound.

### B. R⁷ is a methyl group.

Reaction of 1-benzyloxycarbonyl-2-tetrahydropyranyloxymethyl-1,2,5,6-tetrahydropyridine with bromoform under basic conditions, as in Section XXXVII, gives 3-benzyloxycarbonyl-7,7-dibromo-2-tetrahydropyranyloxymethyl-3-azabicyclo[4.1.0]heptane, which can be further converted into the desired compound by applying methods described in Section XXXVII.

### 1,6,7-R⁶,R⁹,R⁷-Trisubstituted-3-azabicyclo[4.1.0]heptanes (LVIII)

### A. R⁷ is a methyl group.

Addition of benzylamine to 1-tetrahydropyranyloxy-3-buten-2-one, followed by Witting olefination of the ketone with ethylidene triphenylphosphorane, provides 5-benzylamino-3-tetrahydropyranyloxymethyl-2-pentene. Amide formation with monoethyl malonate, followed by diazo transfer and rhodium-catalyzed cycloaddition, can be carried out as described in Section XXVIII to provide the ethyl ester of 3-benzyl-7-methyl-2-oxo-6-tetrahydropyranyloxymethyl-3-azabicyclo[4.1.0]heptane-1-carboxylic acid. This compound can be further processed as in Section XXVIII.

### B. R⁹ is a methyl group.

Addition of benzylamine to methyl vinyl ketone, followed by Peterson olefination of the ketone with ethyl 2-trimethylsilylacetate and base, gives an unsaturated ester which can be reduced with diisobutylaluminum hydride. The resulting primary alcohol is protected as its tetrahydropyranyloxy derivative, to give tetrahydropyranyl-protected 5-benzylamino-3-methyl-pent-2-en-1-ol. Amide formation and cycloaddition as described in Section XXVIII then provides the ethyl ester of 3-benzyl-6-methyl-2-oxo-7-tetrahydropyranyloxymethyl-3-azabicyclo[4.1.0]heptane-1-carboxylic acid. This can be processed into the desired derivative using chemistry outlined in Section XXVIII.

### C. R⁶ is a methyl group.

Addition of benzylamine to 1-tert-butyldimethylsilyloxy-3-buten-2-one, followed by Peterson olefination of the ketone with ethyl 2-trimethylsilylacetate and base, gives an unsaturated ester which can be reduced with diisobutylaluminum hydride. The resulting primary alcohol can be protected as its tetrahydropyranyloxy derivative. Amide formation, cycloaddition and lithium aluminum hydride reduction, as described in Section XXVIII, then gives 3-benzyl-6-tert-butyldimethylsilyloxymethyl-1-hydroxymethyl-7-tetrahydropyranyloxymethyl-3-azabicyclo[4.1.0]heptane. Reduction of the primary alcohol to a methyl group at position 1 can be carried out using the methodology described in Section VI. Subsequent removal of the tert-butyldimethylsilyl protecting group at position 6 can then be effected using tetra-n-butyl ammonium fluoride in tetrahydrofuran solution. The resulting 3-benzyl-6-hydroxymethyl-1-methyl-7-tetrahydropyranyloxymethyl-3- azabicyclo[4.1.0]heptane can be transformed into the desired compound using the chemistry in Sections XI and VIII.

### 4,5,7-R³,R⁵,R⁷-Trisubstituted-3-azabicyclo[4.1.0]heptanes (LXX)

### A. R³ is a methyl group.

1-Benzyloxycarbonyl-1,6-dihydro-3(2H)-pyridinone can be cyclopropanated with ethyl diazoacetate under the influence of molybdenum hexacarbonyl, to give the ethyl ester of 3-benzyloxycarbonyl-5-oxo-3-azabicyclo[4.1.0]heptane-7-carboxylic acid. Treatment of this compound with base, such as lithium hexamethyldisilazide or potassium tert-butoxide, followed by methyl iodide, serves to introduce a methyl group at the 4-position. Wittig reaction and further processing of this compound as in Section XXXIV delivers the desired trisubstituted compound.

### B. R⁵ is a methyl group.

Deprotonation of the ethyl ester of 3-benzyloxycarbonyl-5-oxo-3-azabicyclo[4.1.0]heptane-7-carboxylic acid with a strong base, such as lithium hexamethyldisilazide or potassium tert-butoxide, followed by quenching of the enolate with formaldehyde, gives a primary alcohol which can be protected as its tetrahydropyranyloxy derivative. The resulting ethyl 3-benzyloxycarbonyl-5-oxo-4-tetrahydropyranyloxymethyl-3-azabicyclo[4.1.0]heptane-7-carboxylate is subjected to olefination with base and methyltriphenylphosphonium bromide. Catalytic hydrogenation of the double bond, followed by reintroduction of the benzyloxycarbonyl group, gives the ethyl ester of 3-benzyloxycarbonyl-5-methyl-4-tetrahydropyranyloxymethyl-3-azabicyclo[4.1.0]heptane-7-carboxylic acid, which can be further elaborated as in Section XI.

### C. R⁷ is a methyl group.

1-Benzyloxycarbonyl-5-hydroxy-1,2,5,6-tetrahydropyridine can be transformed into 3-benzyloxycarbonyl-7-bromo-7-methyl-5-hydroxy-3-azabicyclo[4.1.0]heptane using methods described in Section XXXVII. Reaction with tri-(n-butyl)tin hydride then yields the debrominated compound. Oxidation of the alcohol to the ketone with pyridinium chlorochromate or a Swern oxidation provides 3-benzyloxycarbonyl-7-methyl-5-oxo-3-azabicyclo[4.1.0]heptane. Deprotonation, quenching with formaldehyde, and protection as the tetrahydropyranyl derivative as described in Section B above, yields 3-benzyloxycarbonyl-7-methyl-5-oxo-4-tetrahydropyranyloxymethyl-3-azabicyclo[4.1.0]heptane. Transformation of the ketone to the homologated carboxylic acid can be effected as described in Section XXXIV. The resulting 3-benzyloxycarbonyl-7-methyl-4-tetrahydropyranyloxymethyl-3-azabicyclo[4.1.0]heptane-5-carboxylic acid can be converted as in Section XI to give the desired substituents.

The pharmaceutically acceptable acid addition salts of compounds (I) are prepared in a conventional manner by treating a solution or suspension of the free base (I) with about one chemical equivalent of a pharmaceutically acceptable acid. Conventional concentration and recrystallization techniques are employed in isolating the salts. Illustrative of suitable acids are acetic, lactic, succinic, maleic, tartaric, citric, gluconic ascorbic benzoic, methanesulfonic, p-toluenesulfonic, cinnamic, fumaric, phosphonic, hydrochloric, hydrobromic, hydroiodic, sulfamic, and sulfonic acid.

The pharmaceutically acceptable cationic salts of compounds (I) may be prepared by conventional methods from the corresponding acids, e.g. by reaction with about one equimolar amount of a base. These cationic salts do not increase the toxicity of the compound toward animal organisms. Examples of suitable cationic salts are those of alkali metals such as sodium or potassium, alkaline earth metals such as magnesium or calcium, and ammonium or organic amines such as diethanolamine or N-methylglucamine.

The novel compounds of formula I and the pharmaceutically acceptable acid addition salts thereof are useful in the treatment of bacterial infections of broad spectrum, particularly the treatment of gram-positive bacterial strains.

The compounds of the invention may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture. with excipients, or in the form of elixirs or suspensions containing flavoring or coloring agents. In the case of animals, they are advantageously contained in an animal feed or drinking water in a concentration of 5-5000 ppm, preferably 25-500 ppm. They can be injected parenterally, for example, intramuscularly, intravenously or subcutaneously. For parenteral administration, they are best used in the form of a sterile aqueous solution which can contain other solutes, for example, enough salt or glucose to make the solution isotonic. In the case of animals, compounds can be administered intramuscularly or subcutaneously at dosage levels of about 0.1-50 mg/kg/day, advantageously 0.2-10 mg/kg/day given in a single daily dose or up to 3 divided doses.

The invention also provides pharmaceutical compositions comprising an antibacterially effective amount of a compound of the formula (I) together with a pharmaceutically acceptable diluent or carrier.

The compounds of the invention can be administered to humans for the treatment of bacterial diseases by either the oral or parenteral routes, and may be administered orally at dosage levels of about 0.1 to 500 mg/kg/day, advantageously 0.5-50 mg/kg/day given in a single dose or up to 3 divided doses. For intramuscular or intravenous administration, dosage levels are about 0.1-200 mg/kg/day, advantageously 0.5-50 mg/kg/day. While intramuscular administration may be a single dose or up to 3 divided doses, intravenous administration can include a continuous drip. Variations will necessarily occur depending on the weight and condition of the subject being treated and the particular route of administration chosen as will be known to those skilled in the art.

The antibacterial activity of the compounds of the invention is shown by testing according to the Steer's replicator technique which is a standard in vitro bacterial testing method described by E. Steers et al., Antibiotics and Chemotherapy, 9, 307 (1959).

The temperatures are in degrees Celsius in the following preparations and examples.

### Example A

### 1. 5-Benzyl-1,3a,4,5,6,6a-hexahydro-4,6-dioxopyrrolo [3,4-c]pyrazole-3-carboxylic acid, ethyl ester

Ethyl diazoacetate (13 g, 114 mmol) in diethyl ether (100 ml) was added dropwise to a solution of N-benzylmaleimide (10 g, 53 mmol) in diethyl ether (250 ml). The resulting mixture was allowed to stir for 18 hours; the solvent was then removed in vacuo, and the resulting residue partitioned between methylene chloride and water. The organic layer was dried over sodium sulfate, filtered and concentrated to provide the title product as a white solid, mp 145-146° with decomposition (16 g, 53 mmol, 100% yield). ¹H NMR (CDCl₃): 7.31 (m, 5H), 7.02 (bs, 1H), 4.89 (dd, J=11, 2 Hz, 1H), 4.65 (s, 2H), 4.55 (d, J=10 Hz, 1H), 4.36 (q, J=7 Hz, 2H), 1.37 (t, J=7 Hz, 3H).

### 2. [1α,5α,6α]-3-Benzyl-3-azabicyclo[3.1.0]hexane-2,4-dione-6-carboxylic acid, ethyl ester

The title compound of Preparation E.1. (99 g, 0.33 mol) was thermolyzed in a 185° oilbath; after 1.5 hours, the reaction was cooled to room temperature and the product recrystallized from diethyl ether to provide the title product as a white solid, mp 100-101° (31.2 g, 114 mol, 35% yield). ¹H NMR (CDCl₃): 7.29 (s, 5H), 4.50 (s, 2H), 4.17 (q, J=7 Hz, 2H), 2.86 (d, J=3 Hz, 2H), 2.28 (t, J=3 Hz, 1H), 1.26 (t, J=7 Hz, 3H).

### 3. [1α,5α,6α]-3-Benzyl-6-hydroxymethyl-3-azabicyclo[3.1.0]hexane

A solution of ethyl 3-benzyl-3-azabicyclo[3.1.0]hexane-2,4-dione-6-carboxylate (2.73 g, 10 mmol) was added to a suspension of lithium aluminum hydride (1.5 g, 40 mmol) in tetrahydrofuran (250 ml). The resulting mixture was heated to reflux for 28 hours. The reaction mixture was quenched with saturated aqueous ammonium chloride (2 ml) and filtered; the filtrate was concentrated in vacuo to provide the title product as a colorless oil (1.69 g, 8.3 mmol, 83% yield). ¹H NMR (CDCl₃): 7.27 (m, 5H), 3.58 (s, 2H), 3.43 (d, J=7 Hz, 2H), 2.96 (d, J=8 Hz, 2H), 2.35 (bd, J=9 Hz, 2H), 1.58 (m, 1H), 1.28 (s, 2H).

### Example B

### 1. [1α,5α,6α]-6-Hydroxymethyl-3-azabicyclo[3.1.0]hexane

[1α,5α,6α]-3-Benzyl-6-hydroxymethyl-3-azabicyclo[3.1.0]hexane (2.5 g, 12 mmol) was dissolved in methanol (200 ml), treated with palladium hydroxide on carbon (20% palladium content, 500 mg) and stirred under 1 atmosphere of hydrogen for 4.5 hours. The reaction mixture was filtered, and concentrated in vacuo; the residue was mixed with acetonitrile and allowed to crystallize. Filtration provided the title product as an amorphous white solid, mp 98-100° (1.16 g, 10.2 mmol, 85% yield). ¹H NMR (CDCl₃): 3.49 (d, J=7 Hz, 2H), 2.98 (d, J=11 Hz, 2H), 2.85 (bd, J=12 Hz, 2H), 1.67 (bs, 2H), 1.33 (m, 2H), 0.89 (m, 1H).

### 2. [1α,5α,6α]-3-Benzyloxycarbonyl-6-hydroxymethyl-3-azabicyclo[3.1.0]hexane

The title compound of Preparation B.1 (1.0 g, 8.8 mmol) was dissolved in dioxane (40 ml) and water (40 ml) and treated with sodium bicarbonate (3 g, 36 mmol) and benzyl chloroformate (1.3 ml, 9.1 mmol). After 30 minutes, the reaction mixture was extracted with ethyl acetate; the combined organic layers were dried over sodium sulfate, filtered and concentrated to provide the title product as an oil (2.15 g., 8.7 mmol, 99% yield). ¹H NMR (CDCl₃): 7.32 (bs, 5H), 5.08 (s, 2H), 3.65 (m, 2H), 3.46 (m, 4H), 1.45 (m, 2H), 0.91 (m, 1H).

### 3. [1α,5α,6α]-3-Benzyloxycarbonyl-3-azabicyclo[3.1.0]hexane-6-carboxylic acid

A solution of the title compound of Preparation B.2 (2.1 g, 8.5 mmol) in acetone (50 ml) was treated dropwise with Jones' reagent until an orange color persisted. Isopropanol was then added to quench excess oxidant, and the resulting mixture was partitioned between water and methylene chloride. The organic layer was dried over sodium sulfate, filtered and concentrated to provide the title product as an oil (2.08 g, 8.0 mmol, 94% yield). ¹H NMR (CDCl₃): 7.32 (bs, 5H), 5.08 (s, 2H), 3.72 (m, 2H), 3.50 (bs, 2H), 2.13 (bs, 2H), 1.47 (t, J=3 Hz, 1H).

### 4. [1α,5α,6α]-3-Benzyloxycarbonyl-6-tert-butoxycarbonylamino-3-azabicyclo[3.1.0]hexane

Diphenylphosphoryl azide (865 »l, 4 mmol), triethylamine (1.1 ml, 8 mmol) and the title compound of Preparation B.3. (1.0 g, 3.83 mmol) were dissolved in t-butanol (45 ml) and heated to reflux for 18 hours. The solvent was then removed in vacuo, and the residue partitioned between water and ethyl acetate. The combined organic layers were dried over sodium sulfate and concentrated to provide a residue which was purified by column chromatography (eluant: 40% ethyl acetate in hexane). The title product was obtained as an oil (772 mg, 2.3 mmol, 60% yield). ¹H NMR (CDCl₃): 7.31 (s, 5H), 5.06 (s, 2H), 4.65 (bs, 1H), 3.70 (m, 2H), 3.46 (m, 2H), 2.26 (bs, 1H), 1.67 (bs, 2H), 1.41 (s, 9H).

### 5. [1α,5α,6α]-6-tert-Butoxycarbonylamino-3-azabicyclo[3.1.0]hexane

A solution of the title compound of Preparation B.4. (58 mg, 0.17 mmol) was treated with palladium on carbon (10% by weight, 60 mg) and ammonium formate (60 mg, 1 mmol) and heated to 65° for 15 minutes. The reaction mixture was then filtered through Super-cel and the filtrate concentrated in vacuo to provide the title product as a solid (28 mg, 0.14 mmol, 82% yield). ¹H NMR (CDCl₃): 4.65 (bs, 1H), 3.14 (d, J=12 Hz, 2H), 2.93 (m, 2H), 2.30 (bs, 1H), 1.59 (bs, 2H), 1.44 (s, 9H).

### Example C

### 1. [1α,5β,6α]-3-Benzyloxycarbonyl-5-hydroxy-3-azabicyclo[4.1.0]heptane

A flask containing samarium metal (2.7 g, 18.0 mmol) was flame-dried, then charged with tetrahydrofuran (40 ml). A tetrahydrofuran solution (30 ml) of mercuric chloride (467 mg, 1.72 mmol) was added and the mixture was stirred for ten minutes. After addition of 1-benzyloxycarbonyl-5-hydroxy-1,2,5,6-tetrahydropyridine, the flask was cooled to -78°C, and chloroiodomethane (1.25 ml, 17.2 mmol) was added dropwise. The mixture was stirred at room temperature overnight, quenched with saturated aqueous K₂CO₃ solution, and extracted with ether. The ether layer was washed with brine, dried over MgSO₄, and concentrated in vacuo to give a yellow oil. This crude material was chromatographed on neutral alumina-activity I (eluant: 50% ethyl acetate/hexane), providing the title compound as a colorless liquid (750 mg, 3.0 mmol, 81% yield).

¹H NMR (CDCl₃): 7.34 (m, 5H), 5.10 (s, 2H), 4.21 (bs, 1H), 3.68 (d, J=13.2 Hz, 1H), 3.59 (dd, J=13.4, 5.2 Hz, 1H), 3.36 (dd, J=13.9, 4.9 Hz, 1H), 3.16 (dd, J=13.9, 5.6 Hz, 1H), 1.44 (m, 1H), 1.32 (bm, 1H), 0.65 (m, 1H), 0.49 (q, J=5.2 Hz, 1H).

### 2. [1α,5β,6α]-3-Benzyl-5-hydroxy-3-azabicyclo[4.1.0]heptane

To a solution of the compound of Step 1 (3.55 g, 14.36 mmol) in ethanol (150 ml) was added ammonium formate (2.71 g, 43.1 mmol), followed by addition of palladium on activated carbon (10% palladium content, 456 mg, 4.3 mmol). The mixture was stirred at room temperature for 23 hours, then filtered. The filtrate was concentrated on a rotary evaporator to afford the secondary amine (1.62 g, 14.3 mmol, 100% yield).

To a solution of the above-mentioned secondary amine in methanol (150 ml) was added benzaldehyde (1.6 ml, 15.8 mmol) and acetic acid (0.82 ml, 14 mmol) followed by addition of sodium cyanoborohydride (1.6 g, 14 mmol). The mixture was stirred at room temperature overnight. The resulting solution was treated with HCl until the pH value of the solution was about 3. A small amount of gas evolution was observed. The solvent was removed in vacuo, and the residue was treated with aqueous K₂CO₃ solution (pH > 10) and extracted with methylene chloride. The organic layer was washed with brine, dried over magnesium sulfate and evaporated to give the title compound (2.7 g, 13.3 mmol, 93% yield).

¹H NMR (CDCl₃): 7.34 (m, 2H), 7.25 (m, 3H), 4.16 (m, 1H), 3.43 (d, J=13.1Hz, 1H), 3.36 (d, J=13.1 Hz, 1H), 2.62 (d, J=10.8Hz, 1H), 2.52 (dd, J=11.3Hz, 5.3, 1H), 2.28 (dd, J=11.8Hz, 4.5, 1H), 2.14 (dd, J=11.8Hz, 4.6, 1H), 1.68 (bs, 1H), 1.38 (m, 1H), 1.24 (m, 1H), 0.64 (m, 1H), 0.52 (m, 1H).

### 3. 3-Benzyl-3-azabicyclo[4.1.0]heptan-5-one

To a solution of dimethylsulfoxide (4.8 ml, 68.5 mmol) in methylene chloride (150 ml) at -78°C was added oxalyl chloride (2.9 ml, 34 mmol). After 15 minutes, the compound of Step 2 (3.4 g, 17 mmol) was added slowly at this temperature. The mixture was stirred at -78°C for 40 minutes. To this solution was added triethylamine (14.32 ml, 102.8 mmol). The stirring was continued for an additional 5 minutes and the reaction was allowed to warm to room temperature. The reaction mixture was poured into saturated sodium chloride solution and extracted with methylene chloride. The organic layer was washed with brine, dried over magnesium sulfate and evaporated to give the crude material. This was purified by silica gel chromatography (eluant: 15% ethyl acetate/hexane). The title compound was obtained as a viscous oil (2.23 g, 11.1 mmol, 65% yield).

¹H NMR (CDCl₃): 7.32-7.21 (m, 5H), 3.50 (d, J=13.2 Hz, 1H), 3.42 (d, J=13.2 Hz, 1H), 3.26 (d, J=18.5 Hz, 1H), 3.09 (d, J=11.1 Hz, 1H), 2.58 (d, J=18.5 Hz, 1H), 2.45 (dd, J=11.1, 1.3 Hz, 1H), 1.92 (q, J=4.6 Hz, 1H), 1.79 (m, 1H), 1.69 (m, 1H), 1.06 (m, 1H).

### 4. 3-Benzyl-3-azabicyclo[4.1.0]heptan-5-one oxime

A solution of the compound of step 3 (2.23 g, 11.1 mmol) and hydroxylamine hydrochloride (1.0 g, 14.4 mmol) in 80% ethanol (110 ml) was stirred at reflux for 30 minutes. The solvent was removed in vacuo, and the residue was taken up in ether. The organic layer was washed with brine, dried over magnesium sulfate and evaporated to give 3-benzyl-3-azabicyclo[4.1.0]heptan-5-one oxime as a viscous yellow oil (2.28 g, 10.6 mmol, 95% yield).

¹H NMR (CDCl₃, mixture of two isomers): 8.75 (br m, 2H), 7.34-7.17 (m, 10H), 3.77 (d, J=17.8 Hz, 1H), 3.47 (2 doublets, J=13.2 Hz, 2H), 3.46 (2 doublets, J=13.2 Hz, 2H), 3.43 (m, 1H), 3.15 (d, J=14.0 Hz, 1H), 2.96 (d, J=11.0 Hz, 1H), 2.73 (d, J=17.8 Hz, 1H), 2.67 (d, J=14.0 Hz, 1H), 2.47 (dd, J=11.0, 3.6 Hz, 1H), 2.31 (d, J=11.0 Hz, 1H), 2.17 (m, 1H), 1.71 (m, 1H), 1.43 (m, 2H), 1.36 (m, 1H), 1.05 (m, 1H), 0.99 (m, 1H), 0.76 (m, 1H).

### 5. [1α,5α,6α]-3-Benzyl-5-(tert-butoxycarbonyl)amino-3-azabicyclo[4.1.0]heptane

To a solution of the compound of step 4 (2.28 g, 10.6 mmol) in tetrahydrofuran (50 ml) was added a solution of lithium aluminum hydride in tetrahydrofuran (60.6 mmol). The mixture was heated to reflux for 2 hours and, after being cooled to room temperature, was quenched with ethyl acetate (11.6 ml) followed by water (2 ml), aqueous NaOH (15% solution, 6.9 ml) and water (6.9 ml). The resulting precipitate was removed by filtration; the filtrate was diluted with saturated aqueous sodium bicarbonate and extracted with chloroform. The organic layer was washed with brine, dried over magnesium sulfate and evaporated to give the title compound as a viscous yellow oil (1.95 g, 9.65 mmol, 91% yield). This was carried on to the title compound without purification, via one of two routes:

### a) Via di-t-butyl dicarbonate and triethylamine.

To a solution of 3-benzyl-5-amino-3-azabicyclo[4.1.0]heptane (1.95 g, 9.6 mmol) and di-t-butyl dicarbonate (2.3 g, 10.5 mmol) in dioxane (90 ml) and water (10 ml) was added triethylamine (1.6 ml, 11.5 mmol). The mixture was stirred at room temperature for 5 hours, diluted with saturated sodium bicarbonate and extracted with methylene chloride. The organic layer was washed with brine, dried over magnesium sulfate and evaporated to give a yellow oil. This oil was chromatographed on silica gel (eluant: 30% ethyl acetate/hexane) to afford the title compound (1.3 g, 4.3 mmol, 45% yield) from the fraction with high R_{f} value (R_{f} 0.82, 30% ethyl acetate/hexane). The fraction with low R_{f} value (R_{f} 0.68, 30% ethyl acetate/hexane) provided the [1α,5β,6α] isomer (0.56 g, 1.85 mmol, 19% yield).

¹H NMR for title compound (CDCl₃): 7.31-7.19 (m, 5H), 5.24 (d, J=8.1 Hz, 1H), 3.92 (bs, 1H), 3.38 (d, J=13.2 Hz, 1H), 3.31 (d, J=13.2 Hz, 1H). 2.95 (dd, J=11.2, 7.6 Hz, 1H), 2.31 (d, J=11.9 Hz, 1H), 2.13 (m, 2H), 1.41 (s, 9H), 1.09 (m, 1H), 0.95 (m, 1H), 0.63 (m, 1H), 0.26 (m, 1H).

¹H NMR for [1α,5β,6α] isomer (CDCl₃): 7.30-7.20 (m, 5H), 4.70 (bd, 1H), 4.10 (m, 1H), 3.42 (d, J=13.1 Hz, 1H), 3.34 (d, J=13.1 Hz, 1H), 2.61 (m, 1H), 2.51 (m, 1H), 2.31 (dd, J=11.9, 4.9 Hz, 1H), 2.11 (dd, J=11.9, 3.5 Hz, 1H), 1.40 (s, 9H), 1.31 (m, 1H), 1.17 (m, 1H), 0.47 (m, 2H).

### b) Via di-t-butyl dicarbonate and sodium hydroxide.

To a solution of 3-benzyl-5-amino-3-azabicyclo[4.1.0]heptane (518 mg, 2.56 mmol) and di-t-butyl dicarbonate (671 mg, 3.58 mmol) in dioxane (15 ml) was added powdered sodium hydroxide (143 mg) followed by addition of water (5 ml). The mixture was stirred for 1 hour, diluted with water and extracted with ether. The ether layer was washed with brine, dried over magnesium sulfate and evaporated to give an off-white solid, which was chromatographed on silica gel (eluant 30% ethyl acetate/hexane) to afford the title compound as a white solid (187 mg, 0.619 mmol, 24% yield), the [1α,5β,6α] isomer of the title product (144 mg, 0.477 mmol, 19% yield), and a mixture of the title compound and its isomer (263 mg, 0.87 mmol, 34% yield).

### 6. [1α,5α,6α]-5-(tert-Butoxycarbonyl)amino-3-azabicyclo[4.1.0]heptane

To a solution of the title compound of step 5 (1.3 g, 4.3 mmol) in ethanol (50 ml) was added ammonium formate (0.81 g, 12.9 mmol) followed by palladium on activated carbon (10% palladium content, 0.136 g, 1.29 mmol). The mixture was stirred at room temperature for 2 hours, and then filtered. The filtrate was concentrated in vacuo to afford the title compound as a white solid (830 mg, 3.9 mmol, 91% yield).

¹H NMR (CD₃OD): 3.60 (m, 1H) 3.10 (dd, J=13.1, 5.7 Hz, 1H), 2.83 (d, J=13.1 Hz, 1H), 2.61 (dd, J=13.1, 4.7 Hz, 1H), 2.27 (dd, J=13.1, 7.1 Hz, 1H), 1.43 (s, 9H), 0.99 (m, 1H), 0.89 (m, 1H), 0.69 (m, 1H), 0.30 (q, J=5.4 Hz, 1H).

### Example D

### 1. [1α,5β,6α]-5-(tert-Butoxycarbonyl)amino-3-azabicyclo[4.1.0]heptane

To a solution of [1α,5β,6α]-3-benzyl-5-(tert-butoxycarbonyl)amino-3-azabicyclo[4.1.0]heptane, obtained as the minor isomer from Preparation 0.5, (800 mg, 2.64 mmol) in ethanol (50 ml) was added ammonium formate (500 mg, 7.92 mmol) followed by palladium on activated carbon (10% palladium content, 837 mg, 0.79 mmol). The mixture was stirred at room temperature for 1.5 hours, then filtered. The filtrate was concentrated in vacuo to afford 570 mg of the title compound as a waxy yellow solid (570 mg, > 100% weight recovery).

¹H NMR (CDCl₃): 4.80 (bm, 1H), 4.01 (m, 1H), 3.11 (m, 2H), 2.85 (m, 2H), 2.33 (m, 1H), 1.42 (s, 9H), 1.33 (m, 1H), 1.19 (m, 1H), 0.57 (m, 1H), 0.45 (m, 1H).

### Example E

### 1. [1α,6α,7α]-3-Benzyloxycarbonyl-3-azabicyclo[4.1.0]heptane-7-carboxylic acid, ethyl ester

A solution of benzyl 1,2,5,6-tetrahydropyridine-1-carboxylate (20 g, 92 mmol) in methylene chloride (92 ml) was treated with rhodium acetate (1.2 g, 5.5 mmol). A solution of ethyl diazoacetate (31.5 g, 276 mmol) in methylene chloride (8.6 ml) was then added over 22 hours, via syringe pump. After completion of the addition, the reaction mixture was filtered through celite; concentration of the filtrate provided the title compound, which was used in step 2 without purification.

¹H NMR (CDCl₃): 7.32-7.23 (m, 5H), 5.09 (s, 2H), 4:08) (q, J=7.3Hz, 2H), 3.96 (d, J=13.8 Hz, 1H), 3.55 (dd, J=13.8, 4.1 Hz, 1H), 3.45 (bm, 1H), 3.01 (m, 1H), 1.96 (m, 1H), 1.78-1.66 (bm, 3H), 1.45 (t, J=4.3Hz, 1H), 1.23 (t, J=7.3 Hz, 3H).

### 2. [1α,6α,7α]-3-Benzyloxycarbonyl-3-azabicyclo[4.1.0]heptane-7-carboxylic acid

The title compound of step 1 was dissolved in aqueous dioxane (20% by volume, 200 ml). Powdered sodium hydroxide (38 g) was added, and the mixture was stirred at 85°C overnight. After being cooled to room temperature, the solution was extracted with ether. The aqueous layer was acidified with sodium bisulfate to a pH of 2 and extracted with methylene chloride. The methylene chloride layers were washed with brine, dried over magnesium sulfate and concentrated to afford the title compound (13.09 g, 47.5 mmol, crude). This material was utilized in the next reaction step without purification.

¹H NMR (CDCl₃): 7.32-7.23 (m, 5H), 5.09 (s, 2H), 3.96 (d, J=13.8 Hz, 1H), 3.76 (m, 1H), 3.56 (dd, J=13.8, 3.9 Hz, 1H), 3.47 (m, 1H), 3.02 (m, 2H), 1.96 (m, 1H), 1.75 (m, 1H), 1.46 (t, J=3.9 Hz, 1H).

### 3. [1α,6α,7α]-3-Benzyloxycarbonyl-7-(tert-butoxycarbonyl)amino-3-azabicyclo[4.1.0]heptane

A mixture of the compound of step 2 (13.09 g, 47.5 mmol) and triethylamine (7.28 ml, 52.2 mmol) in acetone (150 ml) was cooled to 0°C; ethyl chloroformate (5.4 ml, 57.0 mmol) was added dropwise. The mixture was stirred at 0°C for 30 minutes. A solution of sodium azide (30.85 g, 475 mmol) in water (70 ml) was then added slowly. After an additional 2 hours, the mixture was diluted with water and extracted with ether. The ether layer was washed with brine, dried over magnesium sulfate and concentrated in vacuo to give the acyl azide (7.90 g, 26.3 mmol, crude) which was used directly in the next reaction.

A solution of the acyl azide in toluene (150 ml) was added dropwise to a toluene solution (150 ml) of t-butanol (30 ml) and pyridinium tosylate (9 mg) at 100°C. After completion of the addition, the reaction mixture was maintained at 100°C for 12 hours. The reaction mixture was concentrated in vacuo, and the residue was chromatographed on silica gel (eluant: 20% ethyl acetate/hexane), providing the title compound as a viscous yellow oil, (2.4 g, 6.9 mmol, 7.5% yield from benzyl 1,2,5,6-tetrahydropyridine-1-carboxylate).

¹H NMR (CDCl₃): 7.31 (m, 5H), 5.08 (s, 2H), 4.72 (bs, 1H), 3.88 (bd, J=13.5 Hz, 1H), 3.62 (bm, 1H), 3.32 (bm, 1H), 3.00 (bm, 1H), 2.27 (bm, 1H), 1.94 (m, 1H), 1.77 (m, 1H), 1.41 (s, 9H), 1.19 (m, 2H).

### 4. [1α,6α,7α]-7-(tert-Butoxycarbonyl)amino-3-azabicyclo[4.1.0]heptane

To a solution of the compound of step 3 (2.3 g, 6.6 mmol) in ethanol (100 ml) was added ammonium formate (1.24 g, 19.8 mmol) followed by palladium on activated carbon (10% palladium content, 2.09 g, 1.9 mmol). The mixture was stirred at 60°C for 1 hour and then at room temperature overnight. The reaction mixture was filtered, and the filtrate was concentrated in vacuo to afford the title compound as a viscous, pale yellow oil (1.38 g, 6.51 mmol, 91% yield).

¹H NMR (CD₃OD): 3.20 (dd, J=13.2, 5.8 Hz, 1H), 2.97 (dd, J=13.2, 1.5 Hz, 1H), 2.45 (m, 1H), 2.43 (m, 1H), 2.33 (m, 1H), 1.92 (m, 1H), 1.72 (m, 1H), 1.43 (s, 9H), 1.11 (m, 1H), 1.03 (m, 1H).

The following examples illustrate the invention.

### Example 1

### A. 7-([1α,5α,6α]-6-tert-Butoxycarbonylamino-3-azabicyclo[3.1.0]hex-3-yl)-6-fluoro-1-(2,4-difluorophenyl)-1,4-dihydro-4-oxo-1,8-naphthyridine-3-carboxylic acid, ethyl ester

A solution of [1α,5α,6α]-6-tert-butoxycarbonylamino-3-azabicyclo[3.1.0]hexane (200 mg, 1.01 mmol) in acetonitrile (35 ml) and triethylamine (5 ml) was treated with the ethyl ester of 7-chloro-6-fluoro-1-(2,4-difluorophenyl)-1,4-dihydro-4-oxo-1,8-naphthyridine-3-carboxylic acid (385 mg, 1.01 mmol) and heated to 90° for 18 hours. Removal of solvent in vacuo gave a residue which was partitioned between ethyl acetate and water. The organic layer was treated with activated charcoal, filtered, and concentrated; the residue was then subjected to column chromatography (eluant: 5% methanol in chloroform). The material thus obtained was recrystallized from diethyl ether to give the title product m.p. 256-258°, (296 mg, 0.54 mmol, 54% yield). ¹H NMR (CDCl₃): 8.35 (s, 1H), 8.06 (d, J=13 Hz, 1H), 7.37 (m, 1H), 7.05 (m, 2H), 4.72 (vbs, 1H), 4.37 (q, J=7 Hz, 2H), 3.81 (vbs, 2H), 3.55 (bm, 2H), 2.26 (bs, 1H), 1.78 (bs, 2H), 1.43 (s, 9H), 1.38 (t, J=7 Hz, 2H).

### B. 7-([1α,5α,6α]-6-Amino-3-azabicyclo[3.1.0]-3-yl)-6-fluoro-1-(2,4-difluorophenyl)-1,4-dihydro-4-oxo-1,8-naphthyridine-3-carboxylic acid, hydrochloride salt

The title compound of Example 1.A (250 mg, 0.46 mmol) was dissolved in hydrochloric acid (6N, 20ml) and heated to reflux for 24 hours. The solvent was then removed in vacuo, and the residue trituratad with acetonitrile, washed with diethyl ether and recrystallized from acetonitrile-methanol. The title product was obtained as a pale yellow solid, mp 246° with decomposition (116 mg, 0.26 mmol, 57% yield). ¹H NMR (Methanol-d₄): 8.68 (s, 1H), 7.96 (d, J=13 Hz, 1H), 7.57 (m, 1H), 7.22 (m, 1H), 7.14 (m, 1H), 3.82 (vbs, 2H), 3.62 (vbs, 2H), 2.37 (bs, 1H), 2.03 (bs, 2H).

### Example 2

### A. 7-(1-tert-Butoxycarbonylamino-3-azabicyclo[4.1.0]hept-3-yl)-6-fluoro-1-(2,4-difluorophenyl)-1,4-dihydro-4-oxo-1,8-naphthyridine-3-carboxylic acid, ethyl ester

A solution of 1-tert-butoxycarbonylamino-3-azabicyclo[4.1.0]heptane (200 mg, 0.94 mmol) and the ethyl ester of 7-chloro-6-fluoro-1-(2,4-difluorophenyl)-1,4-dihydro-4-oxo-1,8-naphthyridine-3-carboxylic acid (327 mg, 0.85 mmol) in acetonitrile (12 ml) was heated at reflux for 3 hours. Solvent was removed in vacuo, and the residue was chromatographed on silica gel (eluant: 50% ethyl acetate/hexane) to afford the title product as an off-white solid (423 mg, 0.758 mmol, 88% yield).

¹H NMR (CDCl₃): 8.33 (s, 1H), 8.02 (d, J=13 Hz, 1H), 7.33 (m, 1H), 7.01 (m, 2H), 4.83 (bs, 1H), 4.35 (g, J=7 Hz, 2H), 4.11 (bd, J=13 Hz, 1H), 3.52 (bm, 2H), 3.09 (bm, 1H), 1.99 (bm, 1H), 1.58 (bm, 1H), 1.40 (s, 9H), 1.35 (t, J=7 Hz, 3H), 0.78 (dd, J=12,6 Hz, 1H), 0.42 (t, J=4 Hz, 1H).

### B. 7-(1-Amino-3-azabicyclo[4.1.0]hept-3-yl)-6-fluoro-1-(2,4-difluorophenyl)-1,4-dihydro-4-oxo-1,8-naphthyridine-3-carboxylic acid, hydrochloride salt

A solution of the compound of step A (300 mg, 0.54 mmol) in ethyl acetate (6 ml) and 3N hydrochloric acid (6 ml) was heated to reflux overnight. Solvents were removed in vacuo, and the residue was recrystallized from methanol-acetonitrile to give the title product as a white solid, mp 192°C (decomp.) (155.5 mg, 0.338 mmol, 62% yield).

¹H NMR (DMSO-d₆): 8.86 (s, 1H), 8.16 (d, J=13.7 Hz, 1H), 7.80 (m, 1H), 7.60 (m, 1H), 7.34 (m, 1H), 4.04 (dd, J=13.8, 8.2 Hz, 1H), 3.87 (dd, J=13.8, 9.2 Hz, 1H), 3.40 (m, 1H), 3.18 (m, 1H), 1.97 (m, 1H), 1.46 (m, 2H), 1.10 (m, 1H), 0.64 (m, 1H).

### Example 3

### A. 7-(1-tert-Butoxycarbonylamino-3-azabicyclo[4.1.0]hept-3-yl)-1-cyclopropyl-6-fluoro-1,4-dihydro-4-oxo-quinoline-3-carboxylic acid

According to the procedure of Example 2A, 1-tert-butoxycarbonylamino-3-azabicyclo[4.1.0]heptane (270.0 mg, 1.27 mmol) and 1-cyclopropyl-6,7-difluoro-1,4-dihydro-4-oxo-quinoline-3-carboxylic acid (275.6 mg, 1.03 mmol) were reacted to generate the title compound (304.2 mg, 0.666 mmol, 65%).

¹H NMR (CDCl₃): 8.70 (s, 1H), 7.93 (d, J=13.3 Hz, 1H), 7.28 (m, 1H), 5.03 (bs, 1H), 3.82 (m, 1H), 3.46 (m, 3H), 3.19 (bm, 1H), 2.24 (bm, 1H), 1.93 (bm, 1H), 1.63 (bm, 1H), 1.43 (s, 9H), 1.37 (m, 2H), 1.16 (bs, 2H), 0.94 (dd, J=9.7, 5.5 Hz, 1H), 0.80 (t, J=6.0 Hz, 1H).

### B. 7-(1-Amino-3-azabicyclo[4.1.0]hept-3-yl)-1-cyclopropyl-6-fluoro-1,4-dihydro-4-oxo-quinoline-3-carboxylic acid, hydrochloride salt

According to the procedure of Example 2B, the compound of Step A (287.2 mg, 0.63 mmol) was converted with hydrochloric acid to provide the title compound, mp 235°C (152.4 mg, 0.387 mmol, 62% yield).

### Example 4

### A. 7-(1-tert-Butoxycarbonylamino-3-azabicyclo[4.1.0]hept-3-yl)6-fluoro-1-(2,4-difluorophenyl)-1,4-dihydro-4-oxo-quinoline-3-carboxylic acid, ether ester

According to the procedure of Example 2A, 1-tert-butoxycarbonylamino-3-azabicyclo[4.1.0]heptane (270.0 mg, 1.27 mmol) and 6,7 difluoro-1-(2,4-difluorophenyl)-1,4-dihydro-4-oxo-quinoline-3-carboxylic acid, ethyl ester (463.6 mg, 1.27 mmol) were reacted to generate the tile compound (333.3 mg, 0.59 mmol, 47% yield).

¹H NMR (CDCl₃): 8.24 (s, 1H), 7.92 (d, J=14 Hz, 1H), 7.54 (m, 1H), 7.13 (m, 2H), 6.03 (m, 1H), 4.99 (bs, 1H), 4.31 (q, J=7 Hz, 2H), 3.46 (m, 1H), 3.14 (m, 2H), 2.86 (m, 1H). 2.09 (bm, 1H), 1.77 (m, 1H), 1.38 (m, 13H), 0.84 (dd, J=9, 6 Hz, 1H), 0.71 (m, 1H).

### B. 7-(1-Amino-3-azabicyclo[4.1.0]hept-3-yl)-6-fluoro-1-(2,4-difluorophenyl)-1,4-dihydro-4-oxo-quinoline-3-carboxylic acid, hydrochloride salt

According to the procedure of Example 2B, the compound of Step A (333.3 mg, 0.59 mmol) was converted with hydrochloric acid to provide the title product, mp 223°C (decomp), (128.5 mg, 0.276 mmol, 47% yield).

¹H NMR (DMSO-d₆): 8.84 (s, 1H), 7.98 (d, J=13.5 Hz, 1H), 7.93 (m, 1H), 7.75 (m, 1H), 7.46 (m, 1H), 6.22 (d, J=7.3 Hz, 1H), 3.62 (d, J=12.3 Hz, 1H), 3.40 (dd, J=12.3, 3 Hz, 1H), 3.15 (m, 1H), 2.93 (m, 1H), 2.10 (m, 1H), 1.63 (m, 1H), 1.52 (m, 1H), 1.14 (dd, J=10.4, 5.7 Hz, 1H), 0.71 (m, 1H).

### Example 5

### A. 7-([1α,5α,6α]-5-tert-Butoxycarbonylamino-3-azabicyclo[4.1.0]hept-3-yl)-6-fluoro-1-(2,4-difluorophenyl)-1,4-dihydro-4-oxo-1,8-naphthyridine-3-carboxylic acid, ethyl ester

According to the procedure of Example 2A, [1α,5α,6α]-5-tert-butoxycarbonylamino-3-azabicyclo[4.1.0]heptane (122 mg, 0.57 mmol) and the ethyl ester of 7-chloro-6-fluoro-1-(2,4-difluorophenyl)-1,4-dihydro-4-oxo-1,8-naphthyridine-3-carboxylic acid (218 mg, 0.57 mmol) were reacted to generate the title product (205 mg, 0.367 mmol, 64% yield).

¹H NMR (CDCl₃): 8.36 (s, 1H), 8.09 (d, J=13.8 Hz, 1H), 7.37 (m, 1H), 7.05 (m, 2H), 4.75 (m, 1H), 4.36 (q, J=7 Hz, 2H), 3.87 (m, 2H), 3.46 (m, 2H), 3.20 (m, 1H), 1.43 (s, 9H), 1.36 (t, J=7 Hz, 3H), 1.08 (m, 2H), 0.73 (m, 1H), 0.24 (m, 1H).

### B. 7-([1α,5α,6α]-5-Amino-3-azabicyclo[4.1.0]hept-3-yl)-6-fluoro-1-(2,4-difluorophenyl)-1,4-dihydro-4-oxo-1,8-naphthyridine-3-carboxylic acid, hydrochloride salt

According to the procedure of Example 2B, the compound of Step A (155 mg, 0.27 mmol) was converted with hydrochloric acid to provide the title product, mp 200-210°C (decomp) (50.1 mg, 0.11 mmol, 40% yield).

¹H NMR (D₂O): 8.83 (bs, 1H), 7.88 (bm, 1H), 7.60 (bm, 1H), 7.29 (bm, 2H), 3.9-3.6 (m, 5H), 1.38 (bm, 1H), 1.24 (bm, 1H), 0.92 (bm, 1H), 0.42 (bm, 1H).

### Example 6

### A. 7-([1α,5α,6α]-5-tert-Butoxycarbonylamino-3-azabicyclo[4.1.0]hept-3-yl)-1-cyclopropyl-6-fluoro-1,4-dihydro-4-oxo-1,8-naphthyridine-3-carboxylic acid, ethyl ester

According to the procedure of Example 2A, [1α,5α,6α]-5-tert-butoxycarbonylamino-3-azabicyclo[4.1.0]heptane (150 mg, 0.7 mmol) and the ethyl ester of 7-chloro-1-cyclopropyl-6-fluoro-1,4-dihydro-4-oxo-1,8-naphthyridine-3-carboxylic acid (217.3 mg, 0.7 mmol) were reacted to generate the title product (230 mg, 0.47 mmol, 67% yield).

¹H NMR (CDCl₃): 8.47 (s, 1H), 8.06 (d, J=13.2 Hz, 1H) 5.33 (bs, 1H), 4.35 (q, J=7.3 Hz, 2H), 4.20 (m, 1H), 4.11 (m, 1H), 3.79 (m, 2H), 3.55-3.35 (m, 2H), 1.41 (s, 9H), 1.37 (t, J=7.3 Hz, 3H), 1.21 (m, 4H), 0.98 (m, 2H), 0.81 (m, 1H), 0.34 (m, 1H).

### B. 7-([1α,5α,6α]-5-Amino-3-azabicyclo(4.1.0]hept-3-yl)-1-cyclopropyl-6-fluoro-1,4-dihydro-4-oxo-1,8-naphthyridine-3-carboxylic acid, mesylate salt

According to the procedure of Example 2B, the compound of Step A (220 mg, 0.45 mmol) was converted with methanesulfonic acid in dioxane (15 ml) and water (15 ml) to provide the title compound, mp > 260°C (153.8 mg, 0.339 mmol, 75% yield).

¹H NMR (D₂O): 8.58 (s, 1H), 7.72 (d, J=12.6 Hz, 1H), 4.33 (bm, 1H), 4.08-3.84 (m, 5H), 2.81 (s, 3H), 1.55 (m, 1H), 1.33 (bs, 3H), 1.07 (bs, 3H), 0.60 (bs, 1H).

### Example 7

### A. 7-([1α,5α,6α]-5-tert-Butoxycarbonylamino-3-azabicyclo[4.1.0]hept-3-yl)-1-cyclopropyl-6-fluoro-1,4-dihydro-4-oxo-quinoline-3-carboxylic acid

According to the procedure of Example 2A, [1α,5α,6α]-5-tert-butoxycarbonylamino-3-azabicyclo[4.1.0]heptane (187.8 mg, 0.88 mmol) and 1-cyclopropyl-6,7-difluoro-1,4-dihydro-4-oxo-quinoline-3-carboxylic acid (210 mg, 0.79 mmol) were reacted to generate the title product, mp 167°C (195 mg, 0.426 mmol, 48% yield).

### B. 7-([1α,5α,6α]-5-Amino-3-azabicyclo[4.1.0]hept-3-yl)-1-cyclopropyl-6-fluoro-1,4-dihydro-4-oxo-quinoline-3-carboxylic acid, hydrochloride salt

According to the procedure of Example 2B, the compound of step A (195 mg, 0.43 mmol) was converted with hydrochloric acid to provide the title product, mp 210°C (decomp.) (113.4 mg, 0.289 mmol, 67% yield).

¹H NMR (D₂O): 8.53 (bs, 1H), 7.47 (m, 2H), 4.00 (bs, 1H), 3.88 (m, 1H), 3.68-3.40 (m, 3H), 3.21 (m, 1H), 1.62 (m, 1H), 1.44 (m, 2H), 1.37 (m, 1H), 1.18 (m, 2H), 1.09 (m, 1H), 0.73 (m, 1H).

### Example 8

### A. 7-([1α,5α,6α]-5-tert-Butoxycarbonylamino-3-azabicyclo[4.1.0]hept-3-yl)-1-cyclopropyl-6,8-difluoro-1,4-dihydro-4-oxo-quinoline-3-carboxylic acid

According to the procedure of Example 2A, [1α,5α, 6α]-5-tert-butoxycarbonylamino-3-azabicyclo[4.1.0]heptane (200 mg, 0.94 mmol) and 1-cyclopropyl-6,7,8-trifluoro-1,4-dihydro-4-oxo-quinoline-3-carboxylic acid (313 mg, 0.94 mmol) were reacted to generate the title product (290 mg, 0.61 mmol, 65% yield).

¹H NMR (CDCl₃): 8.74 (s, 1H), 7.84 (d, J=11.6 Hz, 1H), 4.98 (m, 1H), 4.04 (m, 1H), 3.93 (m, 1H), 3.70 (dd, J=12.3, 5.6 Hz, 1H), 3.40 (d, J=12.3 Hz, 1H), 3.32 (m, 1H), 2.89 (m, 1H), 1.39 (s, 9H), 1.24 (m, 2H), 1.12 (m, 2H), 1.04 (m, 1H), 0.79 (m, 1H), 0.51 (m, 1H), 0.29 (m, 1H).

### B. 7-([1α,5α,6α]-5-Amino-3-azabicyclo[4.1.0]hept-3-yl)-1-cyclopropyl-6,8-difluoro-1,4-dihydro-4-oxo-quinoline-3-carboxylic acid, mesylate salt

According to the procedure of Example 2B, the compound of step A (290 mg, 0.61 mmol) was converted with methanesulfonic acid in dioxane (10 ml) and water (10 ml) to provide the title product, mp > 250°C (51.1 mg, 0.11 mmol, 18% yield).

¹H NMR (D₂O-NaOH): 8.50 (s, 1H), 7.66 (d, J=12.4 Hz, 1H), 4.01 (m, 1H), 3.68 (m, 1H), 3.45 (d, J=11.6 Hz, 1H), 3.30 (d, J=11.6 Hz, 1H), 3.22 (m, 1H), 2.83 (s, 3H), 2.79 (m, 1H), 1.23 (m, 2H), 1.10 (m, 2H), 0.98 (m, 1H), 0.74 (m, 1H), 0.46 (m, 1H), 0.22 (m, 1H).

### Example 9

### A. 7-([1α,5α,6α]-5-tert-Butoxycarbonylamino-3-azabicyclo[4.1.0]hept-3-yl)-5-amino-1-cyclopropyl-6,8-difluoro-1,4-dihydro-4-oxo-quinoline-3-carboxylic acid

According to the procedure of Example 2A, [1α,5α,6α-5-tert-butoxycarbonylamino-3-azabicyclo[4.1.0]heptane (110 mg, 0.52 mmol) and 5-amino-1-cyclopropyl-6,7,8-trifluoro-1,4-dihydro-4-oxo-quinoline-3-carboxylic acid (140 mg, 0.46 mmol) were reacted in dimethylsulfoxide to generate the title product (220 mg, 0.45 mmol, 98% yield).

¹H NMR (DMSO-d₆): 8.48 (s, 1H), 7.25 (bs, 1H), 7.10 (d, J=7 Hz, 1H), 4.00 (m, 1H), 3.72 (m, 1H), 3.61 (bd, J=10 Hz, 1H), 3.47 (d, J=12 Hz, 1H), 2.76 (t, J=10 Hz, 1H), 1.38 (s, 9H), 1.16 (m, 1H), 0.97 (m, 1H), 0.69 (m, 1H), 0.35 (m, 1H).

### A. 7-([1α,5α,6α]-5-Amino-3-azabicyclo[4.1.0]hept-3-yl)-5-amino-1-cyclopropyl-6,8-difluoro-1,4-dihydro-4-oxo-quinoline-3-carboxylic acid, hydrochloride salt

According the the procedure of Example 2B, the compound of step A (220 mg, 0.45 mmol) was converted with hydrochloric acid to provide the title product, mp > 238°C (76.5 mg, 0.18 mmol, 40% yield).

¹H NMR (DMSO-d₆/D₂O): 8.50 (s, 1H), 3.99 (m, 1H), 3.62 (m, 1H), 3.47 (m, 2H), 3.01 (m, 2H), 1.32 (m, 1H), 1.07 (m, 5H), 0.81 (m, 1H), 0.53 (m, 1H).

### Example 10

### A. 7-([1α,5β,6α]-5-tert-Butoxycarbonylamino-3-azabicyclo[4.1.0]hept-3-yl)-6-fluoro-1-(2,4-difluorophenyl)-1,4-dihydro-4-oxo-1,8-naphthyridine-3-carboxylic acid, ethyl ester

According to the procedure of Example 2A, [1α,5β,6α]-5-tert-butoxycarbonylamino-3-azabicyclo[4.1.0]heptane (212 mg., 1.0 mmol) and the ethyl ester of 7-chloro-6-fluoro-1-(2,4-difluorophenyl)-1,4-dihydro-4-oxo-1,8-naphthyridine-3-carboxylic acid (363.3 mg, 0.95 mmol) were reacted to generate the title product (389 mg, 0.70 mmol, 73% yield).

¹H NMR (CDCl₃): 8.35 (s, 1H), 8.08 (d, J=13 Hz, 1H), 7.35 (m, 1H), 7.05 (m, 2H), 4.58 (m, 1H), 4.36 (q, J=7 Hz, 2H), 4.05 (m, 1H), 3.80 (m, 1H), 3.45 (m, 1H), 3.30 (m, 1H), 1.44 (bs, 10H), 1.38 (t, J=7 Hz, 3H), 1.22 (m, 1H), 0.54 (m, 1H), 0.26 (m, 1H).

### B. 7-([1α,5β,6α]-5-Amino-3-azabicyclo[4.1.0]hept-3-yl)-6-fluoro-1-(2,4-difluorophenyl)-1,4-dihydro-4-oxo-1,8-naphthyridine-3-carboxylic acid, hydrochloride salt

According to the procedure of Example 2B, the compound of step A (383.4 mg, 0.68 mmol) was converted with hydrochloric acid to provide the title product, mp > 200°C (173.1 mg, 0.377 mmol, 55% yield).

¹H NMR (D₂O): 8.85 (s, 1H), 7.95 (d, J=12.8 Hz, 1H), 7.60 (m, 1H), 7.32 (m, 2H), 4.03 (m, 1H), 3.96-3.73 (m, 2H), 3.53 (m, 2H), 1.55 (m, 1H), 1.46 (m, 1H), 0.84 (m, 1H), 0.56 (m, 1H).

### Example 11

### A. 7-([1α,5β,6α]-5-tert-Butoxycarbonylamino-3-azabicyclo[4.1.0]hept-3-yl)-1-cyclopropyl-6-fluoro-1,4-dihydro-4-oxo-quinoline-3-carboxylic acid

According to the procedure of Example 2A, [1α,5β,6α]-5-tert-butoxycarbonylamino-3-azabicyclo[4.1.0]heptane (133 mg, 0.62 mmol) and 1-cyclopropyl-6,7-difluoro-1,4-dihydro-4-oxo-quinoline-3-carboxylic acid (164 mg, 0.62 mmol) were reacted to generate the title product (99.6 mg, 0.218 mmol, 35% yield).

¹H NMR (CDCl₃): 8.72 (s, 1H), 7.96 (d, J=13.3 Hz, 1H), 7.38 (d, J=7.2 Hz, 1H), 4.82 (bd, J=7.6 Hz, 1H), 4.28 (m, 1H), 3.58 (m, 3H), 3.30 (m, 1H), 3.12 (m, 1H), 1.44 (m, 13H), 1.15 (m, 2H), 0.71 (m, 1H), 0.62 (m, 1H).

### B. 7-([1α,5β,6α]-5-Amino-3-azabicyclo[4.1.0]hept-3-yl)-1-cyclopropyl-6-fluoro-1,4-dihydro-4-oxo-quinoline-3-carboxylic acid, hydrochloride salt

According to the procedure of Example 2B, the compound of step A (99 mg, 0.21 mmol) was converted with hydrochloric acid to provide the title product, mp 252°C (decomp.) (32 mg, 0.081 mmol, 38% yield).

¹H NMR (DMSO-d₆): 8.71 (s, 1H), 8.39 (bs, 2H), 7.97 (d, J=13 Hz, 1H), 7.62 (bs, 1H), 4.0-3.2 (m, 6H), 1.57 (m, 2H), 1.41 (m, 2H), 1.24 (m, 2H), 1.00 (m, 1H), 0.81 (m, 1H).

### Example 12

### A. 7-([1α,6α,7α]-7-tert-Butoxycarbonylamino-3-azabicyclo[4.1.0]hept-3-yl)-6-fluoro-1-(2,4-difluorophenyl)-1,4-dihydro-4-oxo-1,8-naphthyridine-3-carboxylic acid, ethyl ester

According to the procedure of Example 2A, (1α,6α,7α]-7-tert-butoxycarbonylamino-3-azabicyclo[4.1.0]heptane (300 mg, 1.41 mmol) and the ethyl ester of 7-chloro-6-fluoro-1-(2,4-difluorophenyl)-1,4-dihydro-4-oxo-1,8-naphthyridine-3- carboxylic acid (535.3 mg, 1.40 mmol) were reacted to generate the title product (780 mg 1.39 mmol, 99%).

¹H NMR (CDCl₃): 8.40 (s, 1H), 8.12 (d, J=13 Hz, 1H), 7.43 (m, 1H), 7.09 (m, 2H), 4.70 (m, 1H), 4.43 (q, J=7 Hz, 2H), 3.92 (d, J=12 Hz, 1H), 3.70 (m, 1H), 3.40 (m, 1H), 3.10 (m, 1H), 2.28 (m, 1H), 1.99 (m, 1H), 1.82 (m, 1H), 1.45 (s, 9H), 1.42 (t, J=7 Hz, 3H), 1.21 (m, 2H).

### B.1 7-([1α,6α,7α]-7-Amino-3-azabicyclo[4.1.0]hept-3-yl)-6-fluoro-1-(2,4-difluorophenyl)-1,4-dihydro-4-oxo-1,8-naphthyridine-3-carboxylic acid, hydrochloride salt

According to the procedure of Example 2B, the compound of step A (283 mg, 0.51 mmol) was converted with hydrochloric acid to provide the title product, mp 204°C (decomp.) (150 mg, 0.322 mmol, 63% yield).

¹H NMR (D₂O): 8.81 (s, 1H), 7.76 (d, J=13.5 Hz, 1H), 7.57 (m, 1H), 7.29 (m, 2H), 4.01 (d, J=14.9 Hz, 1H), 3.81 (bd, J=13.3 Hz, 1H), 3.45 (m, 1H), 3.20 (m, 1H), 2.50 (bs, 1H), 2.05 (m, 1H), 1.79 (m, 1H), 1.61 (bs, 2H).

### B.2 7-([1α,6α,7α]-7-Amino-3-azabicyclo[4.1.0]hept-3-yl)-6-fluoro-1-(2,4-difluorophenyl)-1,4-dihydro-4-oxo-1,8-naphthyridine-3-carboxylic acid, mesylate salt

According to the procedure of Example 2B, the compound of step A (770 mg, 1.37 mmol) was converted with methanesulfonic acid in dioxane (10 ml) and water (10 ml) to provide the title product, mp 219°C (decomp.) (249.5 mg, 0.474 mmol, 35% yield).

¹H NMR (D₂O-NaOH): 8.35 (s, 1H), 7.93 (d, J=13.4, 1H), 7.54 (m, 1H), 7.24 (m, 2H), 3.85-3.60 (m, 2H), 3.33 (m, 1H), 3.06 (m, 1H), 2.85 (s, 3H), 1.95 (m, 2H), 1.63 (m, 1H), 1.00 (bs, 2H).

### Example 13

### A. 7-([1α,6α,7α]-7-tert-Butoxycarbonylamino-3-azabicyclo[4.1.0]hept-3-yl)-1-cyclopropyl-6-fluoro-1,4-dihydro-4-oxo-1,8-naphthyridine-3-carboxylic acid ethyl ester

According to the procedure of Example 2A, [1α,6α,7α]-7-tert-butoxycarbonylamino-3-azabicyclo[4.1.0]heptane (325 mg, 1.53 mmol) and the ethyl ester of 7-chloro-1-cyclopropyl-6-fluoro-1,4-dihydro-4-oxo-1,8-naphthyridine-3- carboxylic acid (402 mg, 1.29 mmol) were reacted to generate the title product (609 mg, 1.25 mmol, 97% yield).

¹H NMR (CDCl₃): 8.44 (s, 1H), 8.02 (d, J=13 Hz, 1H), 4.77 (bs, 1H), 4.35 (q, J=7 Hz, 2H), 4.25 (d, J=13 Hz, 1H), 3.92 (m, 1H), 3.65 (m, 1H), 3.48 (m, 1H), 3.27 (m, 1H), 2.34 (m, 1H), 2.10 (m, 1H), 1.95 (m, 1H), 1.40 (s, 9H), 1.37 (t, J=7 Hz, 3H), 1.26 (m, 2H), 1.19 (m, 2H), 0.99 (m, 2H).

### B 7-([1α,6α,7α]-7-Amino-3-azabicylo[4.1.0]hept-3-yl)-1-cyclopropyl-6-fluoro-1,4-dihydro-4-oxo-1,8-naphthyridine-3-carboxylic acid, hydrochloride salt

According to the procedure of Example 2B, the compound of step A (585 mg, 1.20 mmol) was converted with hydrochloric acid to provide the title product, mp 180°C (decomp.) (265.7 mg, 0.675 mmol, 56% yield).

¹H NMR (DMSO-d₆): 8.58 (s, 1H), 8.40 (bs, 2H), 8.02 (d, J=13.6 Hz, 1H), 4.19 (d, J=14.1 Hz, 1H), 4.06 (dd, J=13.8, 5.2 Hz, 1H), 3.73 (m, 2H), 3.37 (m, 2H), 2.49 (m, 1H), 2.16 (m, 1H), 1.84 (m, 1H), 1.66 (m, 1H), 1.58 (m, 1H), 1.19 (m, 2H), 1.10 (m, 2H).

## Claims (Claims for the following Contracting State(s): AT, BE, CH, DE, DK, FR, GB, IT, LI, LU, NL, SE)

1. A compound of the formula 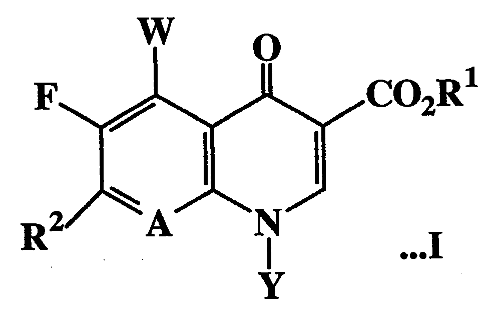 or a pharmaceutically acceptable acid addition salt thereof, wherein
R¹ is hydrogen, a pharmaceutically acceptable cation, or (C₁-C₆) alkyl;
Y, when taken independently, is ethyl, t-butyl, vinyl, cyclopropyl, 2-fluoroethyl, p-fluorophenyl, or o,p-difluorophenyl;
W is hydrogen, fluoro, chloro, bromo, C₁-C₄ alkyl, C₁-C₄ alkoxy, amino or aminomethyl;
A is CH, CF, CC1, COCH₃, C-CH₃, C-CN or N; or
A is carbon and is taken together with Y and the carbon and nitrogen to which A and Y are attached to form a five or six membered ring which may contain oxygen or a double bond, and which may have attached thereto R⁸ which is methyl;
and R² is 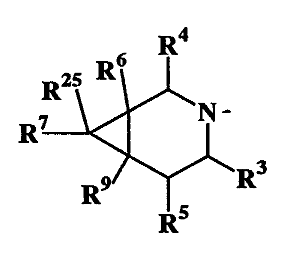 wherein R³, R⁴, R⁵, R⁶, R⁷, R⁹ and R²⁵ are each independently H, CH₃, CH₂NH₂, CH₂NHCH₃ or CH₂NHC₂H₅, and R⁵, R⁶, R⁷, and R⁹ may also be independently be NH₂, NHCH₃ or NHC₂H₅, provided that not more than three of R³, R⁴, R⁵, R⁶, R⁷, R⁹ and R²⁵ are other than hydrogen and if three of these substitutes are not hydrogen, at least one of them is methyl; or wherein R¹ is hydrogen or a pharmaceutically acceptable cation, Y is o,p-difluorophenyl, W is H, A is N and R² is 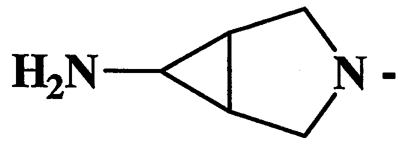 and produgs of those compounds of formula I having free amino groups, these prodrugs comprising an amino acid residue or a polypeptide chain of two or more amino acid residues which are covalently joined through peptide bonds.

2. A compound according to claim 1, characterized in that R¹ and W are each hydrogen.

3. A compound according to claim 1 or 2, characterized in that A is CH or N, or A is carbon and is taken together with Y and the carbon and nitrogen to which A and Y are attached to form a six membered ring as follows:

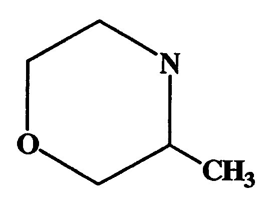

4. A compound according to claim 3, characterized in that A is N.

5. A compound according to anyone of claims 1 to 4, characterized in that one or two of R³, R⁴, R⁵, R⁶, R⁷, R⁹ and R²⁵ are other than hydrogen.

6. A compound according to claim 5, characterized in that one of R³, R⁴, R⁵, R⁶, R⁷ or R⁹ is CH₂NH₂ or CH₂NHCH₃, and, optionally, another of R³, R⁴, R⁵, R⁶, R⁷ or R⁹ is methyl; or one of R⁵, R⁶, R⁷ or R⁹ is NH₂ or NHCH₃ and, optionally, another of R⁵, R⁶, R⁷ or R⁹ or one of R³ or R⁴ is methyl.

7. A compound according to claim 6, characterized in that R⁶, R⁷ or R⁹ is amino and, optionally one of R³, R⁴, R⁵, R⁶, R⁹ or R²⁵ is methyl.

8. A compound according to claim 7, characterized in that R⁷ is amino and optionally, one of R³, R⁴, R⁵, R⁶, R⁹ or R²⁵ is methyl.

9. A pharmaceutical composition comprising a compound of the formula (I) according to any one of claims 1 to 8 or a pharmaceutically acceptable acid addition salt thereof and a pharmaceutically acceptable diluent or carrier.

10. A compound of the formula (I) as claimed in anyone of claims 1 to 8 or a pharmaceutically acceptable acid addition salt thereof for use as a medicament.

11. The use of a compound of the formula (I) as claimed in any one of claims 1 to 8 or of a pharmaceutically acceptable acid addition salt thereof for the manufacture of an antibacterial agent.

12. A compound of formula:

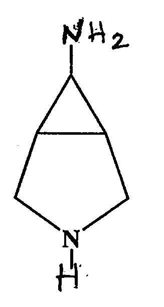

## Claims (Claims for the following Contracting State(s): ES)

1. A process for preparing a compound of the formula 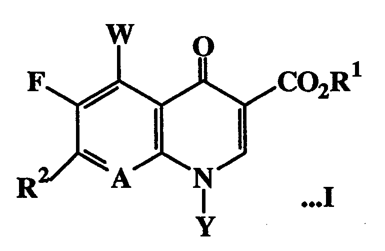 or a pharmaceutically acceptable acid addition salt thereof, wherein
R¹ is hydrogen, a pharmaceutically acceptable cation, or (C₁-C₆) alkyl;
Y, when taken independently, is ethyl, t-butyl, vinyl, cyclopropyl, 2-fluoroethyl, p-fluorophenyl, or o,p-difluorophenyl;
W is hydrogen, fluoro, chloro, bromo, C₁-C₄ alkyl, C₁-C₄ alkoxy, amino or aminomethyl;
A is CH, CF, CC1, COCH₃, C-CH₃, C-CN or N; or
A is carbon and is taken together with Y and the carbon and nitrogen to which A and Y are attached to form a five or six membered ring which may contain oxygen or a double bond, and which may have attached thereto R⁸ which is methyl;
and R² is 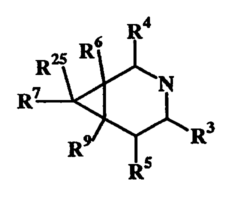 wherein R³, R⁴, R⁵, R⁶, R⁷, R⁹ and R²⁵ are each independently H, CH₃, CH₂NH₂, CH₂NHCH₃ or CH₂NHC₂H₅, and R⁵, R⁶, R⁷, and R⁹ may also be independently be NH₂, NHCH₃ or NHC₂H₅, provided that not more than three of R³, R⁴, R⁵, R⁶, R⁷, R⁹ and R²⁵ are other than hydrogen and if three of these substitutes are not hydrogen, at least one of them is methyl; or wherein R¹ is hydrogen or a pharmaceutically acceptable cation, Y is o,p-difluorophenyl, W is H, A is N and R² is 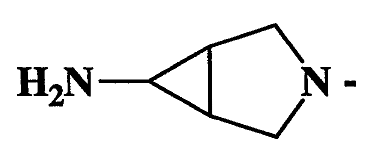 and produgs of those compounds of formula I having free-amino groups, these prodrugs comprising an amino acid residue or a polypeptide chain of two or more amino acid residues which are covalently joined through peptide bonds, characterized by reacting a compound of the formula 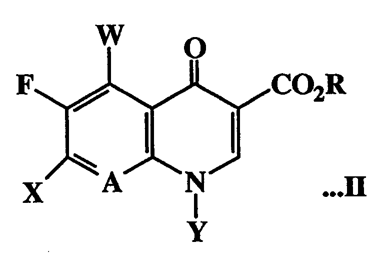 wherein, A, W and Y are as defined above, R¹ is hydrogen or (C₁-C₆)alkyl and X is a leaving group, with a compound of the formula R²H wherein R² is as defined above, and also includes the N-protected groups of NH₂, CH₂NH₂, NHCH₃, CH₂NHCH₃, NHC₂H₅ and CH₂NHC₂H₅, and, if desired, reacting with an acid to form a pharaceutically acceptable acid addition salt, or, when R¹ is hydrogen, reacting with a base to form a compound wherein R¹ is a pharmaceutically acceptable cation, or reacting with an amino acid to form prodrugs of those compounds of formula I having a free amino group.

2. A process according to claim 1, characterized in that R¹ and W are each hydrogen and Y is cyclopropyl or o,p-difluorophenyl.

3. A process according to claim 1 or 2, characterized in that A is CH or N, or A is carbon and is taken together with Y and the carbon and nitrogen to which A and Y are attached to form a six membered ring as follows:

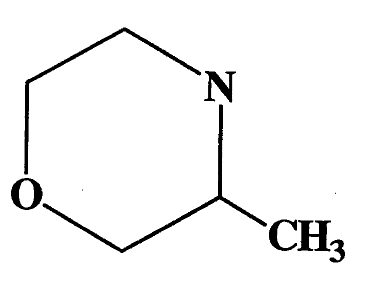

4. A process according to claims 1, 2 or 3, characterized in that one or two of R³, R⁴, R⁵, R⁶, R⁷, R⁹ and R²⁵ are other than hydrogen.

## Claims (Claims for the following Contracting State(s): GR)

1. A process for preparing a compound of the formula 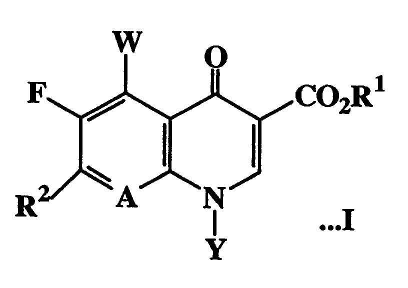 or a pharmaceutically acceptable acid addition salt thereof, wherein
R¹ is hydrogen, a pharmaceutically acceptable cation, or (C₁-C₆) alkyl;
Y, when taken independently, is ethyl, t-butyl, vinyl, cyclopropyl, 2-fluoroethyl, p-fluorophenyl, or o,p-difluorophenyl;
W is hydrogen, fluoro, chloro, bromo, C₁-C₄ alkyl, C₁-C₄ alkoxy, amino or aminomethyl;
A is CH, CF, CC1, COCH₃, C-CH₃, C-CN or N; or
A is carbon and is taken together with Y and the carbon and nitrogen to which A and Y are attached to form a five or six membered ring which may contain oxygen or a double bond, and which may have attached thereto R⁸ which is methyl;
and R² is 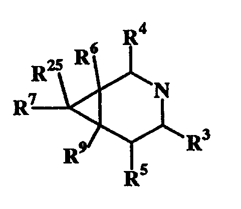 wherein R³, R⁴, R⁵, R⁶, R⁷, R⁹ and R²⁵ are each independently H, CH₃, CH₂NH₂, CH₂NHCH₃ or CH₂NHC₂H₅, and R⁵, R⁶, R⁷, and R⁹ may also be independently be NH₂, NHCH₃ or NHC₂H₅, provided that not more than three of R³, R⁴, R⁵, R⁶, R⁷, R⁹ and R²⁵ are other than hydrogen and if three of these substitutes are not hydrogen, at least one of them is methyl; or wherein R¹ is hydrogen or a pharmaceutically acceptable cation, Y is o,p-difluorophenyl, W is H, A is N and R² is 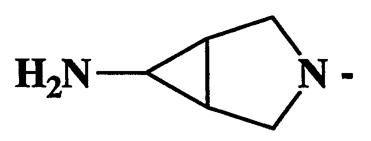 and produgs of those compounds of formula I having free amino groups, these prodrugs comprising an amino acid residue or a polypeptide chain of two or more amino acid residues which are covalently joined through peptide bonds, characterized by reacting a compound of the formula 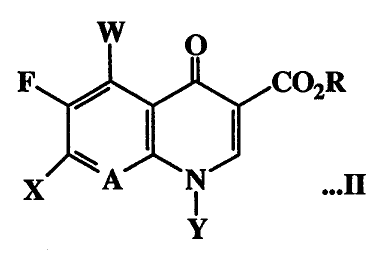 wherein, A, W and Y are as defined above, R¹ is hydrogen or (C₁-C₆)alkyl and X is a leaving group, with a compound of the formula R²H wherein R² is as defined above, and also includes the N-protected groups of NH₂, CH₂NH₂, NHCH₃, CH₂NHCH₃, NHC₂H₅ and CH₂NHC₂H₅, and, if desired, reacting with an acid to form a pharaceutically acceptable acid addition salt, or, when R¹ is hydrogen, reacting with a base to form a compound wherein R¹ is a pharmaceutically acceptable cation, or reacting with an amino acid to form prodrugs of those compounds of formula I having a free amino group.

2. A process according to claim 1, characterized in that R¹ and W are each hydrogen and Y is cyclopropyl or o,p-difluorophenyl.

3. A process according to claim 1 or 2, characterized in that A is CH or N, or A is carbon and is taken together with Y and the carbon and nitrogen to which A and Y are attached to form a six membered ring as follows:

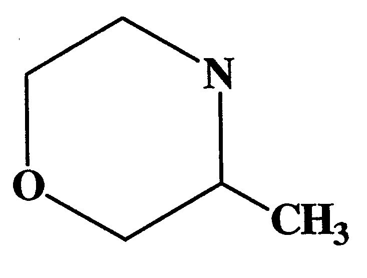

4. A process according to claims 1, 2 or 3, characterized in that one ore two of R³, R⁴, R⁵, R⁶, R⁷, R⁹ and R²⁵ are other than hydrogen.

5. A compound of formula:

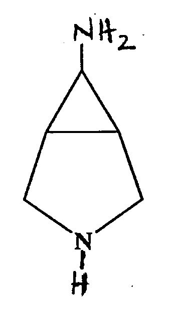

## Patentansprüche (Patentansprüche für folgende(n) Vertragsstaat(en): AT, BE, CH, DE, DK, FR, GB, IT, LI, LU, NL, SE)

1. Verbindung der Formel 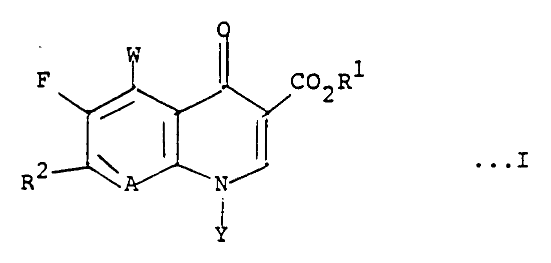 oder ein pharmazeutisch annehmbares Säureadditionssalz davon, worin R¹ Wasserstoff, ein pharmazeutisch annehmbares Kation oder eine (C₁-C₆)-Alkylgruppe ist;
Y, wenn unabhängig, eine Ethyl-, t-Butyl-, Vinyl-, Cyclopropyl-, 2-Fluorethyl-, p-Fluorphenyl- oder o, p-Difluorphenylgruppe ist;
W Wasserstoff, Fluor, Chlor, Brom, eine (C₁-C₄)-Alkyl-, C₁-C₄-Alkoxy-, Amino- oder Aminomethylgruppe ist;
A CH, CF, CCl, COCH₃, C-CH₃, C-CN oder N ist oder A Kohlenstoff ist und zusammen mit Y und dem Kohlenstoff und dem Stickstoff, an die A und Y gebunden sind, einen 5- oder 6-gliedrigen Ring bildet, der Sauerstoff oder eine Doppelbindung enthalten kann, und an den R⁸, das eine Methylgruppe ist, gebunden sein kann, und
R² 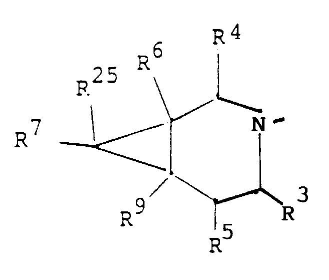 worin R³, R⁴, R⁵, R⁶, R⁷, R⁹ und R²⁵ unabhängig H, CH₃, CH₂-NH₂, CH₂NHCH₃ oder CH₂NHC₂H₅ bedeuten und R⁵, R⁶, R⁷ und R⁹ unabhängig auch NH₂, NHCH₃ oder NHC₂H₅ sein können, mit dem Vorbehalt, daß nicht mehr als 3 der Reste R³, R⁴, R⁵, R⁶, R⁷, R⁹ und R²⁵ etwas anderes als Wasserstoff sind und wenn 3 dieser Substituenten nicht Wasserstoff sind, mindestens einer von ihnen eine Methylgruppe ist; oder worin R¹ Wasserstoff oder ein pharmazeutisch annehmbares Kation ist, Y o,p-Difluorphenyl ist, W H ist, A N ist und R² 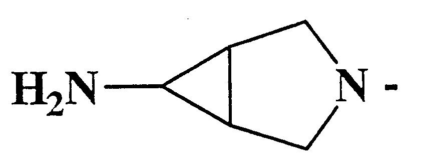 ist, und Prodrugs derjenigen Verbindungen der Formel I, die freie Aminogruppen haben, wobei diese Prodrugs einen Aminosäurerest oder eine Polypeptidkette mit zwei oder mehreren Aminosäureresten umfassen, die kovalent durch Peptidbindungen verbunden sind.

2. Verbindung nach Anspruch 1, dadurch gekennzeichnet, daß R¹ und W jeweils Wasserstoff sind.

3. Verbindung nach Anspruch 1 oder 2, dadurch gekennzeichnet, daß A CH oder N ist oder A Kohlenstoff ist und zusammen mit Y und dem Kohlenstoffatom und dem Stickstoffatom, an die A und Y gebunden sind, einen sechsgliedrigen Ring wie folgt bildet:

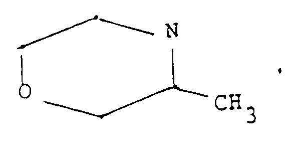

4. Verbindung nach Anspruch 3, dadurch gekennzeichnet, daß A N ist.

5. Verbindung nach einem der Ansprüche 1 bis 4, dadurch gekennzeichnet, daß einer oder zwei der Reste R³, R⁴, R⁵, R⁶, R⁷, R⁹ und R²⁵ eine andere Bedeutung als Wasserstoff haben.

6. Verbindung nach Anspruch 5, dadurch gekennzeichnet, daß einer der Reste R³, R⁴, R⁵, R⁶, R⁷ oder R⁹ CH₂NH₂ oder CH₂NHCH₃ ist und fakultativ ein anderer der Reste R³, R⁴, R⁵, R⁶, R⁷ oder R⁹ Methyl ist; oder einer der Reste R⁵, R⁶, R⁷ oder R⁹ NH₂ oder NHCH₃ ist und fakultativ ein anderer der Reste R⁵, R⁶, R⁷ oder R⁹ oder einer der Reste R³ oder R⁴ Methyl ist.

7. Verbindung nach Anspruch 6, dadurch gekennzeichnet, daß R⁶, R7 oder R⁹ Amino ist und fakultativ einer der Reste R³, R⁴, R⁵, R⁶, R⁹ oder R²⁵ Methyl ist.

8. Verbindung nach Anspruch 7, dadurch gekennzeichnet, daß R⁷ Amino ist und fakultativ einer der Reste R³, R⁴, R⁵, R⁶, R⁹ oder R²⁵ Methyl ist.

9. Pharmazeutische Zusammensetzung, umfassend eine Verbindung der Formel (I) nach einem der Ansprüche 1 bis 8 oder ein pharmazeutisch annehmbares Säureadditionssalze davon und ein pharmazeutisch annehmbares Verdünnungsmittel oder einen Träger.

10. Verbindung der Formel (I) nach einem der Ansprüche 1 bis 8 oder ein pharmazeutisch annehmbares Säureadditionssalz davon zur Verwendung als Arzneimittel.

11. Verwendung einer Verbindung der Formel (I) nach einem der Ansprüche 1 bis 8 oder eines pharmazeutisch annehmbaren Säureadditionssalzes davon zur Herstellung eines antibakteriellen Mittels.

12. Verbindung der Formel

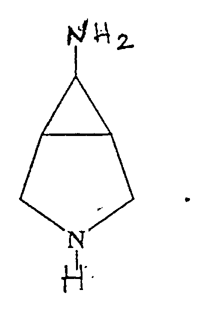

## Patentansprüche (Patentansprüche für folgende(n) Vertragsstaat(en): ES)

1. Verfahren zur Herstellung einer Verbindung der Formel 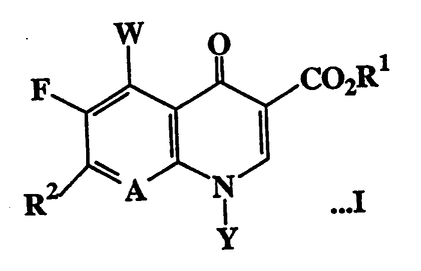 oder eines pharmazeutisch annehmbaren Säureadditionssalzes davon, worin
R¹ Wasserstoff, ein pharmazeutisch annehmbares Kation oder eine (C₁-C₆)-Alkylgruppe ist;
Y, wenn unabhängig, eine Ethyl-, t-Butyl-, Vinyl-, Cyclopropyl-, 2-Fluorethyl-, p-Fluorphenyl- oder o, p-Difluorphenylgruppe ist;
W Wasserstoff, Fluor, Chlor, Brom, eine (C₁-C₄)-Alkyl-, C₁-C₄-Alkoxy-, Amino- oder Aminomethylgruppe ist;
A CH, CF, CCl, COCH₃, C-CH₃, C-CN oder N ist oder
A Kohlenstoff ist und zusammen mit Y und dem Kohlenstoff und dem Stickstoff, an die A und Y gebunden sind, einen 5- oder 6-gliedrigen Ring bildet, der Sauerstoff oder eine Doppelbindung enthalten kann, und an den R⁸, das eine Methylgruppe ist, gebunden sein kann, und
R² 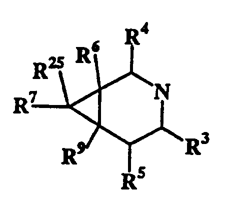 worin R³, R⁴, R⁵, R⁶, R⁷, R⁹ und R²⁵ unabhängig H, CH₃, CH₂-NH₂, CH₂NHCH₃ oder CH₂NHC₂H₅ bedeuten und R⁵, R⁶, R⁷ und R⁹ unabhängig auch NH₂, NHCH₃ oder NHC₂H₅ sein können, mit dem Vorbehalt, daß nicht mehr als 3 der Reste R³, R⁴, R⁵, R⁶, R⁷, R⁹ und R²⁵ etwas anderes als Wasserstoff sind und wenn 3 dieser Substituenten nicht Wasserstoff sind, mindestens einer von ihnen eine Methylgruppe ist; oder worin R¹ Wasserstoff oder ein pharmazeutisch annehmbares Kation ist, Y o,p-Difluorphenyl ist, W H ist, A N ist und R² 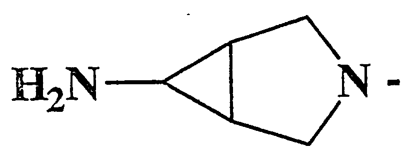 ist, und Prodrugs derjenigen Verbindungen der Formel I, die freie Aminogruppen haben, wobei diese Prodrugs einen Aminosäurerest oder eine Polypeptidkette mit zwei oder mehreren Aminosäureresten umfassen, die kovalent durch Peptidbindungen verbunden sind,
**gekennzeichnet durch** das Umsetzen einer Verbindung der Formel 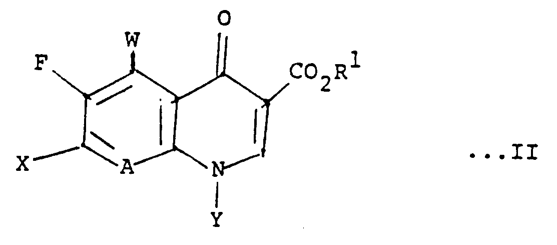 worin A, W und Y wie oben definiert sind, R¹ Wasserstoff oder eine C₁-C₆-Alkylgruppe ist und X eine Abgangsgruppe ist, mit einer Verbindung der Formel R²H, worin R² wie oben definiert ist und auch die N-geschützten Gruppen von NH₂, CH₂NH₂, NHCH₃, CH₂NHCH₃, NHC₂H₅ und CH₂NHC₂H₅ einschließt und, falls gewünscht, Umsetzen mit einer Säure, um ein pharmazeutisch annehmbares Säureadditionssalz zu bilden, oder, wenn R¹ Wasserstoff ist, Umsetzen mit einer Base, um eine Verbindung zu bilden, worin R¹ ein pharmazeutisch annehmbares Kation ist oder mit einer Aminosäure umsetzt, um Prodrugs der Verbindungen der Formel I mit einer freien Aminogruppe zu bilden.

2. Verfahren nach Anspruch 1, **dadurch gekennzeichnet**, daß R¹ und W jeweils Wasserstoff sind und Y eine Cyclopropyl- oder o,p-Difluorphenylgruppe ist.

3. Verfahren nach einem der Ansprüche 1 oder 2, **dadurch gekennzeichnet,** daß A CH oder N ist oder A Kohlenstoff ist und zusammen mit Y und dem Kohlenstoffatom und dem Stickstoffatom,an die A und Y gebunden sind, einen sechsgliedrigen Ring, wie folgt bildet:

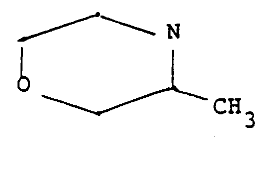

4. Verfahren nach den Ansprüchen 1, 2 oder 3, **dadurch gekennzeichnet**, daß einer oder zwei der Substituenten R³, R⁴, R⁵, R⁶, R⁷, R⁹ und R²⁵ eine andere Bedeutung als Wasserstoff hat/haben.

## Patentansprüche (Patentansprüche für folgende(n) Vertragsstaat(en): GR)

1. Verfahren zur Herstellung einer Verbindung der Formel 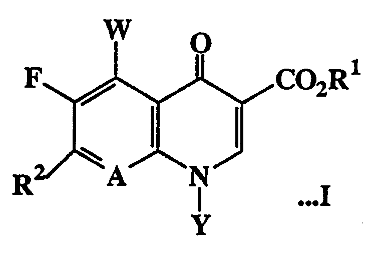 oder eines pharmazeutisch annehmbaren Säureadditionssalzes davon, worin
R¹ Wasserstoff, ein pharmazeutisch annehmbares Kation oder eine (C₁-C₆)-Alkylgruppe ist;
Y, wenn unabhängig; eine Ethyl-, t-Butyl-, Vinyl-, Cyclopropyl-, 2-Fluorethyl-, p-Fluorphenyl- oder o, p-Difluorphenylgruppe ist;
W Wasserstoff, Fluor, Chlor, Brom, eine (C₁-C₄)-Alkyl-, C₁-C₄-Alkoxy-, Amino- oder Aminomethylgruppe ist;
A CH, CF, CCl, COCH₃, C-CH₃, C-CN oder N ist oder
A Kohlenstoff ist und zusammen mit Y und dem Kohlenstoff und dem Stickstoff, an die A und Y gebunden sind, einen 5- oder 6-gliedrigen Ring bildet, der Sauerstoff oder eine Doppelbindung enthalten kann, und an den R⁸, das eine Methylgruppe ist, gebunden sein kann, und
R² 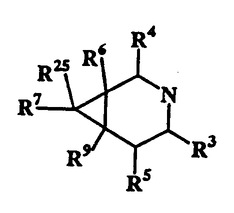 worin R³, R⁴, R⁵, R⁶, R⁷, R⁹ und R²⁵ unabhängig H, CH₃, CH₂-NH₂, CH₂NHCH₃ oder CH₂NHC₂H₅ bedeuten und R⁵, R⁶, R⁷ und R⁹ unabhängig auch NH₂, NHCH₃ oder NHC₂H₅ sein können, mit dem Vorbehalt, daß nicht mehr als 3 der Reste R³, R⁴, R⁵, R⁶, R⁷, R⁹ und R²⁵ etwas anderes als Wasserstoff sind und wenn 3 dieser Substituenten nicht Wasserstoff sind, mindestens einer von ihnen eine Methylgruppe ist; oder worin R¹ Wasserstoff oder ein pharmazeutisch annehmbares Kation ist, Y o,p-Difluorphenyl ist, W H ist, A N ist und R² 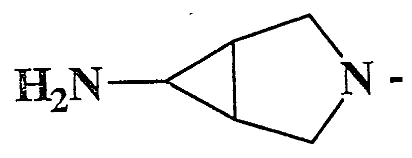 ist, und Prodrugs derjenigen Verbindungen der Formel I, die freie Aminogruppen haben, wobei diese Prodrugs einen Aminosäurerest oder eine Polypeptidkette mit zwei oder mehreren Aminosäureresten umfassen, die kovalent durch Peptidbindungen verbunden sind,
**gekennzeichnet durch** das Umsetzen einer Verbindung der Formel 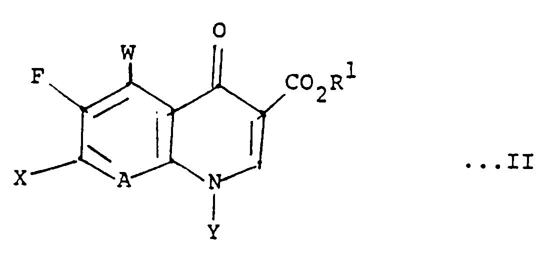 worin A, W und Y wie oben definiert sind, R¹ Wasserstoff oder eine C₁-C₆-Alkylgruppe ist und X eine Abgangsgruppe ist, mit einer Verbindung der Formel R²H, worin R² wie oben definiert ist und auch die N-geschützten Gruppen von NH₂, CH₂NH₂, NHCH₃, CH₂NHCH₃, NHC₂H₅ und CH₂NHC₂H₅ einschließt und, falls gewünscht, Umsetzen mit einer Säure, um ein pharmazeutisch annehmbares Säureadditionssalz zu bilden, oder, wenn R¹ Wasserstoff ist, Umsetzen mit einer Base, um eine Verbindung zu bilden, worin R¹ ein pharmazeutisch annehmbares Kation ist oder mit einer Aminosäure umsetzt, um Prodrugs der Verbindungen der Formel I mit einer freien Aminogruppe zu bilden.

2. Verfahren nach Anspruch 1, **dadurch gekennzeichnet**, daß R¹ und W jeweils Wasserstoff sind und Y eine Cyclopropyl- oder o,p-Difluorphenylgruppe ist.

3. Verfahren nach einem der Ansprüche 1 oder 2, **dadurch gekennzeichnet,** daß A CH oder N ist oder A Kohlenstoff ist und zusammen mit Y und dem Kohlenstoffatom und dem Stickstoffatom,an die A und Y gebunden sind, einen sechsgliedrigen Ring, wie folgt bildet:

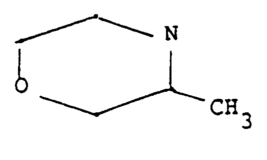

4. Verfahren nach den Ansprüchen 1, 2 oder 3, **dadurch gekennzeichnet**, daß einer oder zwei der Substituenten R³, R⁴, R⁵, R⁶, R⁷, R⁹ und R²⁵ eine andere Bedeutung als Wasserstoff hat/haben.

5. Verbindung der Formel

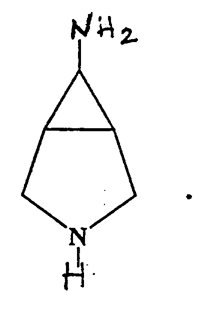

## Revendications (Revendications pour l'(les) Etat(s) contractant(s) suivant(s): AT, BE, CH, DE, DK, FR, GB, IT, LI, LU, NL, SE)

1. Composé de formule 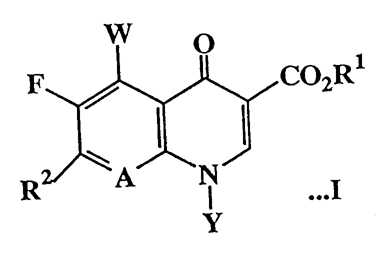 ou un sel d'addition d'acide pharmaceutiquement acceptable de ce composé, formule dans laquelle
R¹ est de l'hydrogène, un cation pharmaceutiquement acceptable ou un groupe alkyle en C₁ à C₆ ;
Y, considéré indépendamment, représente un groupe éthyle, tertio-butyle, vinyle, cyclopropyle, 2-fluoréthyle, p-fluorophényle ou o,p-difluorophényle ;
W est de l'hydrogène, un radical fluoro, chloro, bromo, alkyle en C₁ à C₄, alkoxy en C₁ à C₄, amino ou aminométhyle ;
A est un groupe CH, CF, CCl, COCH₃, C-CH₃, C-CN ou N ; ou bien
A est un atome de carbone et forme, conjointement avec Y et avec le carbone et l'azote auxquels A et Y sont attachés, un noyau pentagonal ou hexagonal qui peut contenir de l'oxygène ou une double liaison, et auquel peut être attaché un groupe R⁸ qui est un groupe méthyle ;
et R² est un groupe 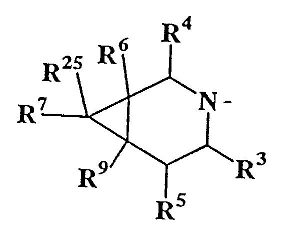 dans lequel R³, R⁴, R⁵, R⁶, R⁷, R⁹ et R²⁵ représentent chacun, indépendamment, H, un groupe CH₃, CH₂NH₂, CH₂NHCH₃ ou CH₂NHC₂H₅, et R⁵, R⁶, R⁷ et R⁹ peuvent aussi représenter indépendamment un groupe NH₂, NHCH₃ ou NHC₂H₅, sous réserve que pas plus de trois de R³, R⁴, R⁵, R⁶, R⁷, R⁹ et R²⁵ représentent autre chose que de l'hydrogène et que, si trois de ces substituants ne représentent pas de l'hydrogène, au moins l'un d'entre eux représente un groupe méthyle ; ou bien R¹ est de l'hydrogène ou un cation pharmaceutiquement acceptable, Y est un groupe o,p-difluorophényle, W représente H, A représente N et R² est un groupe 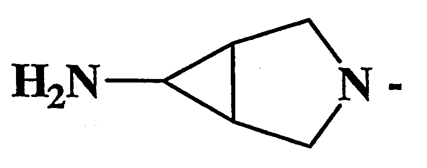 et des promédicaments de ces composés de formule I portant des groupes amino libres, ces promédicaments comprenant un résidu d'aminoacide ou une chaîne polypeptidique de deux ou plus de deux résidus d'aminoacides qui sont liés par covalence par l'intermédiaire de liaisons peptidiques.

2. Composé suivant la revendication 1, caractérisé en ce que R¹ et W représentent chacun de l'hydrogène.

3. Composé suivant la revendication 1 ou 2, caractérisé en ce que A représente CH ou N, ou bien A est du carbone et forme conjointement avec Y et avec les atomes de carbone et d'azote auxquels A et Y sont attachés, un noyau hexagonal de formule suivante :

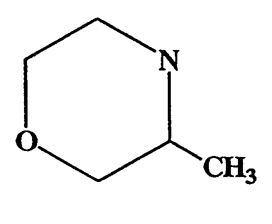

4. Composé suivant la revendication 3, caractérisé en ce que A est un atome d'azote.

5. Composé suivant l'une quelconque des revendications 1 à 4, caractérisé en ce qu'un ou deux de R², R³, R⁴, R⁵, R⁶, R⁷, R⁹ et R²⁵ représentent autre chose que de l'hydrogène.

6. Composé suivant la revendication 5, caractérisé en ce que l'un de R³, R⁴, R⁵, R⁶, R⁷ ou R⁹ est un groupe CH₂NH₂ ou CH₂NHCH₃ et, à titre facultatif, un autre de R³, R⁴, R⁵, R⁶, R⁷ ou R⁹ est un groupe méthyle ; ou bien l'un de R⁵, R⁶, R⁷ ou R⁹ est un groupe NH² ou NHCH₃ et, à titre facultatif, un autre de R⁵, R⁶, R⁷ ou R⁹ ou l'un de R³ ou R⁴ est un groupe méthyle.

7. Composé suivant la revendication 6, caractérisé en ce que R⁶, R⁷ ou R⁹ est un groupe amino et, à titre facultatif, l'un de R³, R⁴, R⁵, R⁶, R⁹ ou R²⁵ est un groupe méthyle.

8. Composé suivant la revendication 7, caractérisé en ce que R⁷ est un groupe amino et, à titre facultatif, l'un de R³, R⁴, R⁵, R⁶, R⁹ ou R²⁵ est un groupe méthyle.

9. Composition pharmaceutique, comprenant un composé de formule (I) suivant l'une quelconque des revendications 1 à 8 ou un sel d'addition d'acide pharmaceutiquement acceptable de ce composé et un diluant ou support acceptable du point de vue pharmaceutique.

10. Composé de formule (I) suivant l'une quelconque des revendications 1 à 8 ou sel d'addition d'acide pharmaceutiquement acceptable de ce composé destiné à être utilisé comme médicament.

11. Utilisation d'un composé de formule (I) suivant l'une quelconque des revendications 1 à 8 ou d'un sel d'addition d'acide pharmaceutiquement acceptable de ce composé pour la production d'un agent antibactérien.

12. Composé de formule :

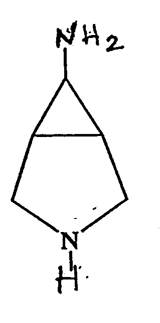

## Revendications (Revendications pour l'(les) Etat(s) contractant(s) suivant(s): ES)

1. Procédé de production d'un composé de formule 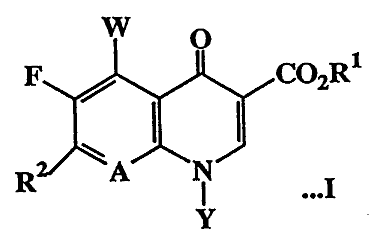 ou d'un sel d'addition d'acide pharmaceutiquement acceptable de ce composé, formule dans laquelle
R¹ est de l'hydrogène, un cation pharmaceutiquement acceptable ou un groupe alkyle en C₁ à C₆ ;
Y, considéré indépendamment, représente un groupe éthyle, tertio-butyle, vinyle, cyclopropyle, 2-fluoréthyle, p-fluorophényle ou o,p-difluorophényle ;
W est de l'hydrogène, un radical fluoro, chloro, bromo, alkyle en C₁ à C₄, alkoxy en C₁ à C₄, amino ou aminométhyle ;
A est un groupe CH, CF, CCl, COCH₃, C-CH₃, C-CN ou N ; ou bien
A est un atome de carbone et forme, conjointement avec Y et avec le carbone et l'azote auxquels A et Y sont attachés, un noyau pentagonal ou hexagonal qui peut contenir de l'oxygène ou une double liaison, et auquel peut être attaché un groupe R⁸ qui est un groupe méthyle ;
et R² est un groupe 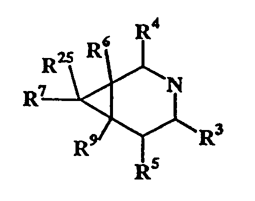 dans lequel R³, R⁴, R⁵, R⁶, R⁷, R⁹ et R²⁵ représentent chacun, indépendamment, H, un groupe CH₃, CH₂NH₂, CH₂NHCH₃ ou CH₂NHC₂H₅, et R⁵, R⁶, R⁷ et R⁹ peuvent aussi représenter indépendamment un groupe NH₂, NHCH₃ ou NHC₂H₅, sous réserve que pas plus de trois de R³, R⁴, R⁵, R⁶, R⁷, R⁹ et R²⁵ représentent autre chose que de l'hydrogène et que, si trois de ces substituants ne représentent pas de l'hydrogène, au moins l'un d'entre eux représente un groupe méthyle ; ou bien R¹ est de l'hydrogène ou un cation pharmaceutiquement acceptable, Y est un groupe o,p-difluorophényle, W représente H, A représente N et R² est un groupe 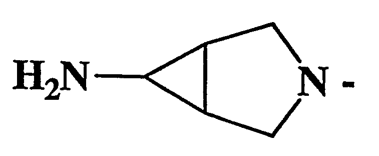 et des promédicaments de ces composés de formule I portant des groupes amino libres, ces promédicaments comprenant un résidu d'aminoacide ou une chaîne polypeptidique de deux ou plus de deux résidus d'aminoacides qui sont liés par covalence par l'intermédiaire de liaisons peptidiques, caractérisé par la réaction d'un composé de formule 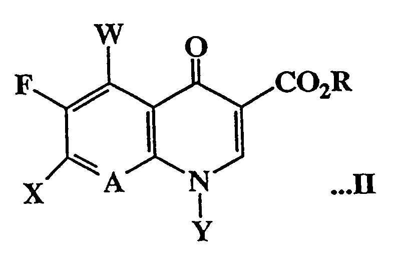 dans laquelle A, W et Y sont tels que définis ci-dessus, R¹ est de l'hydrogène ou un groupe alkyle en C₁ à C₆ et X est un groupe partant, avec un composé de formule R²H dans laquelle R² est tel que défini ci-dessus, y compris les groupes N-protégés de NH₂, CH₂NH₂, NHCH₃, CH₂NHCH₃, NHC₂H₅ et CH₂NHC₂H₅ et, le cas échéant, la réaction avec un acide pour former un sel d'addition d'acide pharmaceutiquement acceptable ou bien, lorsque R¹ est de l'hydrogène, la réaction avec une base pour former un composé dans lequel R¹ est un cation pharmaceutiquement acceptable, ou la réaction avec un aminoacide pour former des promédicaments des composés de formule I portant un groupe amino libre.

2. Procédé suivant la revendication 1, caractérisé en ce que R¹ et W représentent chacun de l'hydrogène et Y est un groupe cyclopropyle ou o,p-difluorophényle.

3. Procédé suivant la revendication 1 ou 2, caractérisé en ce que A représente CH ou N, ou bien A est du carbone et forme, conjointement avec Y et avec le carbone et l'azote auxquels A et Y sont attachés, un noyau hexagonal de formule suivante :

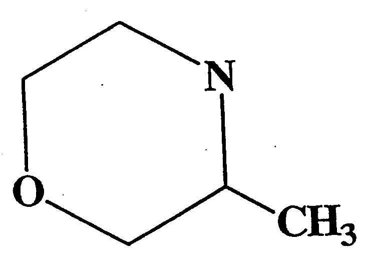

4. Procédé suivant la revendication 1, 2 ou 3, caractérisé en ce qu'un ou deux de R³, R⁴, R⁵, R⁶, R⁷, R⁹ et R²⁵ représentent autre chose que de l'hydrogène.

## Revendications (Revendications pour l'(les) Etat(s) contractant(s) suivant(s): GR)

1. Procédé de production d'un composé de formule 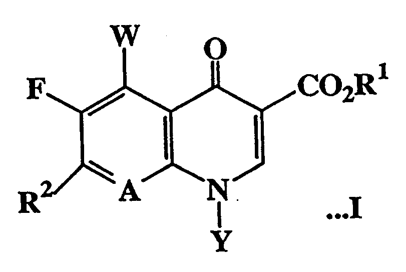 ou d'un sel d'addition d'acide pharmaceutiquement acceptable de ce composé, formule dans laquelle
R¹ est de l'hydrogène, un cation pharmaceutiquement acceptable ou un groupe alkyle en C₁ à C₆ ;
Y, considéré indépendamment, représente un groupe éthyle, tertio-butyle, vinyle, cyclopropyle, 2-fluoréthyle, p-fluorophényle ou o,p-difluorophényle ;
W est de l'hydrogène, un radical fluoro, chloro, bromo, alkyle en C₁ à C₄, alkoxy en C₁ à C₄, amino ou aminométhyle ;
A est un groupe CH, CF, CCl, COCH₃, C-CH₃, C-CN ou N ; ou bien
A est un atome de carbone et forme, conjointement avec Y et avec le carbone et l'azote auxquels A et Y sont attachés, un noyau pentagonal ou hexagonal qui peut contenir de l'oxygène ou une double liaison, et auquel peut être attaché un groupe R⁸ qui est un groupe méthyle ;
et R² est un groupe 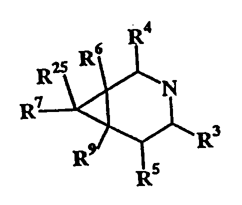 dans lequel R³, R⁴, R⁵, R⁶, R⁷, R⁹ et R²⁵ représentent chacun, indépendamment, H, un groupe CH₃, CH₂NH₂, CH₂NHCH₃ ou CH₂NHC₂H₅, et R⁵, R⁶, R⁷ et R⁹ peuvent aussi représenter indépendamment un groupe NH₂, NHCH₃ ou NHC₂H₅, sous réserve que pas plus de trois de R³, R⁴, R⁵, R⁶, R⁷, R⁹ et R²⁵ représentent autre chose que de l'hydrogène et que, si trois de ces substituants ne représentent pas de l'hydrogène, au moins l'un d'entre eux représente un groupe méthyle ; ou bien R¹ est de l'hydrogène ou un cation pharmaceutiquement acceptable, Y est un groupe o,p-difluorophényle, W représente H, A représente N et R² est un groupe 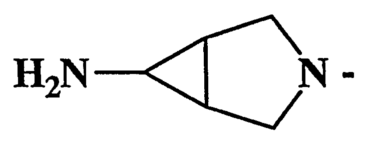 et des promédicaments de ces composés de formule I portant des groupes amino libres, ces promédicaments comprenant un résidu d'aminoacide ou une chaîne polypeptidique de deux ou plus de deux résidus d'aminoacides qui sont liés par covalence par l'intermédiaire de liaisons peptidiques, caractérisé par la réaction d'un composé de formule 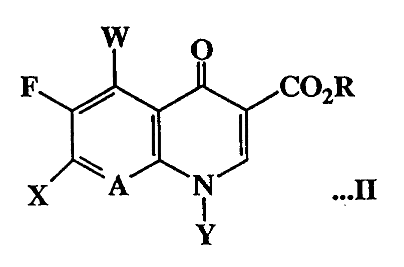 dans laquelle A, W et Y sont tels que définis ci-dessus, R¹ est de l'hydrogène ou un groupe alkyle en C₁ à C₆ et X est un groupe partant, avec un composé de formule R²H dans laquelle R² est tel que défini ci-dessus, y compris les groupes N-protégés de NH₂, CH₂NH₂, NHCH₃, CH₂NHCH₃, NHC₂H₅ et CH₂NHC₂H₅ et, le cas échéant, la réaction avec un acide pour former un sel d'addition d'acide pharmaceutiquement acceptable ou bien, lorsque R¹ est de l'hydrogène, la réaction avec une base pour former un composé dans lequel R¹ est un cation pharmaceutiquement acceptable, ou la réaction avec un aminoacide pour former des promédicaments des composés de formule I portant un groupe amino libre.

2. Procédé suivant la revendication 1, caractérisé en ce que R¹ et W représentent chacun de l'hydrogène et Y est un groupe cyclopropyle ou o,p-difluorophényle.

3. Procédé suivant la revendication 1 ou 2, caractérisé en ce que A représente CN ou N, ou bien A est du carbone et forme, conjointement avec Y et avec le carbone et l'azote auxquels A et Y sont attachés, un noyau hexagonal de formule suivante :

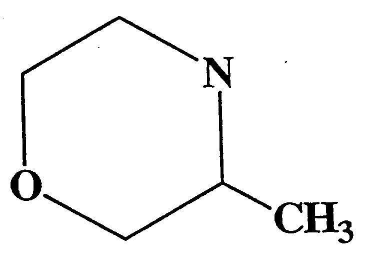

4. Procédé suivant la revendication 1, 2 ou 3, caractérisé en ce qu'un ou deux de R³, R⁴, R⁵, R⁶, R⁷, R⁹ et R²⁵ représentent autre chose que de l'hydrogène.

5. Composé de formule :
